# EUROPÄISCHE PATENTSCHRIFT

(11) **EP 4 422 532 B1**
(45) Veröffentlichungstag und Bekanntmachung des Hinweises auf die Patenterteilung: **02.12.2026**
(21) Anmeldenummer: 22801960.0
(22) Anmeldetag: 07.11.2022
(51) Int. Cl.: A61B 17/74

(54) **VORRICHTUNG ZUM VERSORGEN EINER FRAKTUR**
DEVICE FOR TREATING A FRACTURE
DISPOSITIF DE TRAITEMENT D'UNE FRACTURE

(43) Veröffentlichungstag der Anmeldung: 04.09.2024
(73) Patentinhaber: I.T.S. GmbH, 8301 Lassnitzhöhe (AT)
(72) Erfinder: PRAGER, Ronald, 24796 Bovenau (DE); BÜHREN, Volker, 82418 Murnau (DE)
(74) Vertreter: Wirnsberger & Lerchbaum Patentanwälte OG
(86) Internationale Anmeldenummer: PCT/AT2022/060379
(87) Internationale Veröffentlichungsnummer: WO 2024/098081

(56) Entgegenhaltungen:
- AT-A4- 524 767
- DE-A1- 102019 108 264
- US-A1- 2005 055 024

## Beschreibung

Die Erfindung betrifft eine Vorrichtung zum Versorgen einer Fraktur, insbesondere einer Fraktur eines proximalen Femurs wie beispielsweise einer Schenkelhalsfraktur, aufweisend einen Nagel, welcher in einen intramedullären Kanal eines Knochens einbringbar ist, insbesondere in einen intramedullären Kanal eines Oberschenkelknochenschaftes, wobei der Nagel eine Bohrung aufweist, welche etwa quer zu einer Längsachse des Nagels ausgerichtet ist, wobei eine Koppeleinrichtung, insbesondere eine Knochenschraube, vorgesehen ist, welche derart in die Bohrung einbringbar ist, dass die Koppeleinrichtung die Bohrung beidseits überragt und in der Bohrung entlang einer Querachse in einer Lateralrichtung und einer entgegengesetzt zur Lateralrichtung orientierten Medialrichtung bewegbar ist, und welche Koppeleinrichtung endseitig in einem Knochenteil, insbesondere einem Oberschenkelkopf, fixierbar ist, insbesondere mittels eines an der Koppeleinrichtung vorgesehenen Gewindes, wobei eine Justiereinrichtung vorgesehen ist, welche in unterschiedlichen Positionen relativ zum Nagel fixierbar ist, insbesondere mittels eines Gewindes in der Justiereinrichtung und im Nagel, und wobei die Justiereinrichtung eine erste Anschlagfläche aufweist, welche bei Anordnung der Koppeleinrichtung und der Justiereinrichtung am Nagel eine Bewegbarkeit der Koppeleinrichtung in Lateralrichtung begrenzt, sodass durch eine Änderung der Position der Justiereinrichtung relativ zum Nagel eine laterale Endposition veränderbar ist, bis zu welcher die Koppeleinrichtung relativ zum Nagel in Lateralrichtung bewegbar ist.

Aus dem Stand der Technik, nämlich der AT 524 767 A4, sind Vorrichtungen der eingangs genannten Art bekannt geworden, um insbesondere eine Schenkelhalsfraktur zu versorgen.

Ausschlaggebend für eine rasche Heilung ist dabei eine gewisse Bewegbarkeit zwischen dem Oberschenkelkopf und dem Oberschenkelknochenschaft, weswegen beispielsweise aus dem Dokument US 8,172,841 B2 eine Vorrichtung bekannt geworden ist, welche eine gewisse Bewegbarkeit der Koppeleinrichtung relativ zum Nagel erlaubt. Nach diesem Dokument wird diese Bewegbarkeit mittels einer distal am Nagel angeordneten Madenschraube erreicht, welche in einer Nut der Koppeleinrichtung gleitet.

Es hat sich allerdings gezeigt, dass mit einer derartigen Vorrichtung nicht bei allen Patienten eine optimale Heilung erreicht werden kann.

Hier setzt die Erfindung an. Aufgabe der Erfindung ist es, eine Vorrichtung der eingangs genannten Art anzugeben, welche besser an die Bedürfnisse einzelner Patienten anpassbar ist.

Die Aufgabe wird erfindungsgemäß durch eine Vorrichtung gemäß Anspruch 1 gelöst.

Im Rahmen der Erfindung wurde erkannt, dass nicht für sämtliche Patienten die gleiche Bewegbarkeit der Koppeleinrichtung relativ zum Nagel bzw. ein Spiel in Querrichtung zwischen Koppeleinrichtung und Nagel gleichermaßen günstig ist, sondern abhängig von patientenspezifischen Parametern wie insbesondere einer Größe, einem Gewicht, einer Knochendichte, einer Art der Fraktur und dergleichen, unterschiedliche Beträge vorteilhaft sein können, um welche die Koppeleinrichtung relativ zum Nagel bewegbar ist. Diese die Heilung fördernde Anpassbarkeit der Bewegbarkeit bzw. des Spiels wird erfindungsgemäß auf konstruktiv besonders einfache Weise durch eine in unterschiedlichen Positionen relativ zum Nagel fixierbare Justiereinrichtung erreicht, welche eine Bewegbarkeit der Koppeleinrichtung in Lateralrichtung über eine erste Anschlagfläche formschlüssig begrenzt.

Die Koppeleinrichtung ist üblicherweise mittels eines endseitig vorgesehenen Gewindes mit dem Oberschenkelkopf verbindbar und hierzu in der Regel als Knochenschraube ausgebildet. Dennoch kann die Koppeleinrichtung auch auf jede andere geeignete Art ausgebildet sein, um mit einem Knochenteil wie beispielsweise dem Oberschenkelkopf verbindbar zu sein.

Weiter versteht es sich, dass die erfindungsgemäße Vorrichtung auch zur Versorgung anderer Frakturen als einer Schenkelhalsfraktur eingesetzt werden kann, insbesondere auch zur Versorgung einer Fraktur im Bereich des proximalen Femurs.

Die Querachse steht üblicherweise etwa quer zu einer Längsachse des Nagels, in der Regel unter einem Winkel, unter welchem eine Achse des Oberschenkelhalses zu einer Achse des Oberschenkelknochenschaftes steht, beispielsweise 110° bis 140°.

Die Justiereinrichtung ist vorzugsweise in den Nagel einschraubbar, und zwar bevorzugt distal der Koppeleinrichtung und etwa vorzugsweise unter einem geringen Winkel zur Querachse. Somit kann durch eine Änderung einer Einschraubtiefe der üblicherweise als Justierschraube ausgebildeten Justiereinrichtung ein Spiel der Koppeleinrichtung relativ zum Nagel leicht verändert werden.

Die erste Anschlagfläche ist durch mehrere Anschlagflächen gebildet, welche parallel wirken und somit eine Bewegbarkeit besonders effektiv begrenzen. So hat sich gezeigt, dass bei Einsatz nur einer einzigen ersten Anschlagfläche ein Risiko besteht, dass die Justiereinrichtung entlang der Koppeleinrichtung gleitet und somit die Bewegung weniger als intendiert begrenzt wird. Insbesondere kann vorgesehen sein, dass die erste Anschlagfläche durch zwei oder drei parallel wirkende Anschläge gebildet wird, beispielsweise einen primären, einen sekundären und einen tertiären ersten Anschlag. Dabei kann der primäre Anschlag beispielsweise an einem lateralen Ende der Justiereinrichtung, der sekundäre Anschlag beispielsweise etwa mittig an der Justiereinrichtung und der tertiäre Anschlag im Bereich eines medialen Endes der Justiereinrichtung angeordnet sein.

Günstig ist es, wenn die erste Anschlagfläche zumindest teilweise durch eine Schulter oder einen Kragen, insbesondere einen Justierschraubenkragen, in einem Bereich eines lateralen Endes der Justiereinrichtung gebildet ist. Der Kragen kann als Justierschraubenkragen ausgebildet und an einem lateralen Ende der Justierschraube positioniert sein, um beispielsweise an einer vordefinierten Relativposition von Koppeleinrichtung und Justiereinrichtung an der Koppeleinrichtung anzuliegen und somit eine weitere Bewegung der Koppeleinrichtung relativ zur Justiereinrichtung in Lateralrichtung verhindern.

Bevorzugt ist vorgesehen, dass die erste Anschlagfläche zumindest teilweise durch einen Teil eines Gewindes der Justiereinrichtung gebildet ist, durch welches Gewinde die Justiereinrichtung relativ zum Nagel fixierbar ist. Insbesondere ein mediales Ende des Gewindes kann als Anschlag für eine korrespondierende Fläche der Koppeleinrichtung dienen, um eine Bewegung der Koppeleinrichtung in Lateralrichtung zu begrenzen.

Es hat sich bewährt, dass die erste Anschlagfläche zumindest teilweise durch einen Teil eines Gewindes der Koppeleinrichtung gebildet ist, durch welches Gewinde die Koppeleinrichtung endseitig in einem Knochenteil fixierbar ist. Das Gewinde der Koppeleinrichtung kann somit als Anschlag dienen, indem beispielsweise ein laterales Ende des Gewindes bei einer vordefinierten Relativposition von Justiereinrichtung und Koppeleinrichtung an der Justiereinrichtung anliegt und somit eine weitere Bewegung von Koppeleinrichtung in Lateralrichtung begrenzt.

Bevorzugt ist vorgesehen, dass die Justiereinrichtung mit dem Nagel durch ein Gewinde verbunden ist, wobei eine Gewindesicherung, insbesondere ein Kunststoffteil, welches eine Reibung im Gewinde erhöht, vorgesehen ist, um ein Lösen der Justiereinrichtung zu verhindern. Somit ist ein unbeabsichtigtes Lösen der Justiereinrichtung auf einfache Weise vermieden.

Um Fehler bei einem Einbau der Vorrichtung durch zu weites Einschrauben auf einfache Weise zu verhindern, ist bevorzugt vorgesehen, dass die Justiereinrichtung nur bis zu einer vordefinierten Position in den Nagel einschraubbar ist, wobei dies konstruktiv insbesondere mit einem Schraubenschlüssel, vorzugsweise einem Innensechskantschlüssel, umgesetzt ist, der ab einer vordefinierten Position der Justiereinrichtung keinen Kontakt mehr zu einem korrespondierenden Gegenstück, insbesondere einem Innensechskant, in der Justiereinrichtung herstellt.

Günstig ist es, wenn die Justiereinrichtung eine zweite Anschlagfläche aufweist, welche bei Anordnung der Koppeleinrichtung und der Justiereinrichtung am Nagel eine Bewegbarkeit der Koppeleinrichtung in Medialrichtung begrenzt.

Weiter kann auch vorgesehen sein, dass die Justiereinrichtung unter einem Winkel zur Koppeleinrichtung steht, sodass sich eine Rampe ergibt, welche über den Winkel eine Bewegbarkeit der Koppeleinrichtung definiert. Die Bewegbarkeit der Koppeleinrichtung relativ zum Nagel in Querrichtung kann somit konstruktiv über einen Winkel zwischen Justiereinrichtung und Koppeleinrichtung und noch während einer Operation über eine Position der Justiereinrichtung relativ zum Nagel bzw. eine Einschraubtiefe verändert werden. Der Winkel beträgt beispielsweise 0,8° bis 2°.

Alternativ hierzu könnte natürlich auch vorgesehen sein, dass die mediale Endposition der Koppeleinrichtung relativ zum Nagel durch einen Anschlag am Nagel gebildet wird, welcher ab einer gewissen Position eine weitere Bewegung der Koppeleinrichtung in medialer Richtung verhindert. Beispielsweise kann hierzu an einem lateralen Ende der Koppeleinrichtung, welche an einer lateralen Seite aus dem Nagel ragt, ein Kragen bzw. eine Schulter vorgesehen sein, welche größer ist als ein Durchmesser einer Bohrung im Nagel, durch welche Bohrung die Koppeleinrichtung durch den Nagel geführt wird, sodass der laterale Kragen bzw. die laterale Schulter ab einer vordefinierten Position am Nagel anliegt, und somit einen Anschlag bildet, welcher eine weitere Bewegung der Koppeleinrichtung in medialer Richtung verhindert.

Bevorzugt ist vorgesehen, dass eine Position der Justiereinrichtung relativ zum Nagel entlang einer Justierrichtung veränderbar ist, insbesondere entlang einer Geraden. Dies kann besonders dann auf einfache Weise umgesetzt werden, wenn die Justiereinrichtung mit einem Außengewinde und der Nagel mit einem korrespondierenden Innengewinde ausgebildet sind, wobei eine Achse entlang der Justierrichtung verläuft.

Bevorzugt ist vorgesehen, dass die erste Anschlagfläche und die zweite Anschlagfläche unter unterschiedlichen Winkeln zur Justierrichtung ausgerichtet sind, sodass bei einer Änderung der Position der Justiereinrichtung relativ zum Nagel die laterale Endposition um einen größeren Betrag als die mediale Endposition änderbar ist. Dadurch ist somit nicht nur eine Lage des Spiels der Koppeleinrichtung relativ zum Nagel mittels einer Änderung der Position der Justiereinrichtung relativ zum Nagel veränderbar, sondern auch eine Größe des Spiels. Es kann auch vorgesehen sein, dass sich durch eine Lage der zweiten Anschlagfläche nicht mit einer Position der Justiereinrichtung ändert. Dies ist insbesondere dann der Fall, wenn die zweite Anschlagfläche durch eine Zylindermantelfläche einer als Justierschraube ausgebildeten Justiereinrichtung ausgebildet ist. Es wird dann nur eine Größe des Spiels und eine Lage der ersten Anschlagfläche mit einer Position der Justiereinrichtung bzw. mit einer Einschraubtiefe der Justierschraube verändert.

Besonders bevorzugt ist vorgesehen, dass eine Position der Justiereinrichtung relativ zum Nagel entlang einer Justierrichtung veränderbar ist, wobei die Justierrichtung unter einem

Winkel von 0,1° bis 15°, insbesondere 0,8° bis 2°, zur Querrichtung ausgerichtet ist, wobei vorzugsweise die Justierrichtung und die Querrichtung in derselben Ebene liegen.

Auf diese Weise wird konstruktiv besonders einfach eine Ausbildung erreicht, bei der eine Veränderung der Position der Justiereinrichtung relativ zum Nagel unterschiedliche Auswirkungen auf die Position der ersten Anschlagfläche einerseits und der zweiten Anschlagfläche andererseits hat. So kann die erste Anschlagfläche beispielsweise durch eine zur Justierrichtung etwa normale Fläche der Justiereinrichtung, insbesondere eine Gewindeflanke einer als Justierschraube ausgebildeten Justiereinrichtung, gebildet werden, während die zweite Anschlagfläche durch eine zur Justierrichtung beispielsweise rotationssymmetrisch, insbesondere eine Kegelfläche oder eine konzentrisch zur Justierrichtung verlaufende Zylinderfläche bzw. eine Außenfläche der als Justierschraube gebildeten Justiereinrichtung gebildet werden kann. Bei entsprechend kleinem Winkel zwischen Justierrichtung und Querrichtung ergibt sich somit, dass eine Änderung der Position der Justiereinrichtung in Justierrichtung eine etwa entsprechend große Auswirkung auf die Position der ersten Anschlagfläche und nahezu keine oder gar keine Auswirkung auf eine Position der zweiten Anschlagfläche hat.

Beispielsweise ist dann, wenn die zweite Anschlagfläche durch eine Zylinderfläche der Justiereinrichtung gebildet wird, welche konzentrisch zur Justierrichtung ist, die Justierrichtung unter einem Winkel zur Querrichtung ausgerichtet ist und in der Koppeleinrichtung eine zur Justiereinrichtung korrespondierende Nut unter einem entsprechenden Winkel angeordnet ist, eine Lage der zweiten Anschlagfläche nur durch den Winkel definiert, ändert sich jedoch im Unterschied zur ersten Anschlagfläche nicht bei einer Änderung einer Einschraubtiefe der Justiereinrichtung, wobei die erste Anschlagfläche durch eine Stirnseite oder eine Gewindeflanke der Justiereinrichtung gebildet ist, welche Justiereinrichtung als Justierschraube ausgebildet sein kann. Üblicherweise ist vorgesehen, dass die Justiereinrichtung länger als die Bohrung ausgebildet ist, sodass die Justiereinrichtung in einer die Bohrung beidseitig überragenden Position mit dem Nagel verbindbar ist, wobei die erste Anschlagfläche medial vom Nagel angeordnet ist. Üblicherweise durchsetzt die Justiereinrichtung den Nagel vollständig, beispielsweise an einer Öffnung des Nagels. Die Öffnung, durch welche die Justiereinrichtung durch den Nagel ragt, kann dabei auch mit der Bohrung zusammenfallen, durch welche die Koppeleinrichtung durch den Nagel ragt. Die entsprechende Bohrung kann somit an einem distalen Bereich mit einem Gewinde für die Justiereinrichtung ausgebildet sein.

Koppeleinrichtung und Justiereinrichtung sind üblicherweise etwa länglich, vorzugsweise etwa zylindrisch ausgebildet, zumal entsprechende Bohrungen im Oberschenkelkopf in aller Regel ebenfalls einen zylindrischen Querschnitt aufweisen.

Günstig ist es, wenn die Koppeleinrichtung eine mit der Justiereinrichtung zusammenwirkende und längs der Koppeleinrichtung verlaufende Nut aufweist, sodass eine Rotation der Koppeleinrichtung um die Querachse durch die Justiereinrichtung begrenzt, vorzugsweise verhindert, ist, wenn die Koppeleinrichtung und die Justiereinrichtung mit dem Nagel verbunden sind. Dies ist besonders günstig, weil während einer Heilung einer Schenkelhalsfraktur, wobei der Schenkelhals naturgemäß belastet wird, üblicherweise auch eine Torsionsbelastung auf den Schenkelhals wirkt, welche zu einer unerwünschten Rotation der Koppeleinrichtung relativ zum Nagel führen könnte. Eine solche Rotation um die Querachse wird somit auf effiziente Weise verhindert, wenn eine entsprechende Nut vorgesehen ist, welche mit der Justiereinrichtung derart zusammenwirkt, dass die Justiereinrichtung in die Nut eingreift und somit eine Rotation der Koppeleinrichtung relativ zum Nagel verhindert.

Um eine einfache Konstruktion einerseits und einen besonders guten Kontakt zwischen der Justiereinrichtung und der Koppeleinrichtung andererseits zu erreichen, ist es günstig, wenn die Bohrung zur Aufnahme der Koppeleinrichtung in einem proximalen Bereich und zur Aufnahme der Justiereinrichtung in einem distalen Bereich ausgebildet ist. Es versteht sich, dass die Angaben distal, proximal, lateral und medial sich hier jeweils auf eine bestimmungsgemäße Anordnung der Vorrichtung an einem Knochen eines menschlichen Körpers beziehen, üblicherweise eines Oberschenkelknochens. Bei bestimmungsgemäßer Anwendung ist die Justiereinrichtung somit unterhalb bzw. distal von der Koppeleinrichtung angeordnet.

Günstig ist es, wenn die Koppeleinrichtung eine von einem lateralen Ende bis zu einem medialen Ende ragende Durchgangsbohrung aufweist, in welche ein K-Draht einführbar ist. Somit kann die Koppeleinrichtung besonders genau in einen Schenkelhals eingebracht werden, indem zunächst nur eine Bohrung mit einem geringen Durchmesser in den Schenkelhals eingebracht wird, wonach der K-Draht in diese Bohrung mit geringem Durchmesser eingebracht wird, wonach der K-Draht als Führung für eine Bohrung mit einem größeren, dem Durchmesser der Koppeleinrichtung etwa entsprechendem Durchmesser eingebracht wird, wobei der K-Draht als Führung für diesen größeren Bohrer dient, wonach der größere Bohrer entnommen und die Koppeleinrichtung wiederum mittels des K-Drahtes als Führung in den Schenkelhals eingebracht wird.

Besonders günstig ist es, wenn eine kanülierte Einbringeinrichtung vorgesehen ist, welche alternativ zur Justiereinrichtung mit dem Nagel verbindbar ist, insbesondere anstelle der Justiereinrichtung in die Bohrung des Nagels einführbar ist, sodass durch die kanülierte Einbringeinrichtung Knochenzement oder ein anderes Augmentationsmaterial bis in einen Bereich eines Oberschenkelkopfes einbringbar ist, wenn der Knochennagel in einem Oberschenkelknochenschaft angeordnet und die Einbringeinrichtung mit dem Nagel verbunden ist. Üblicherweise wird der Knochenzement oder das andere Augmentationsmaterial durch eine in die Kanülierung der Einbringeinrichtung, welche als Durchgangsbohrung durch die Einbringeinrichtung ausgebildet sein kann, lösbar und temporär eingebrachte Kanüle eingebracht. Üblicherweise umfasst die Vorrichtung auch eine derartige Kanüle, welche mit der kanülierten Einbringeinrichtung korrespondiert.

Selbstverständlich kann alternativ zu einer Kanüle, welche temporär in die Einbringeinrichtung eingebracht wird, um den Knochenzement oder das andere Augmentationsmaterial durch die Einbringeinrichtung in die erste Knochenbohrung einzubringen, auch vorgesehen sein, dass die Einbringeinrichtung selbst als Kanüle ausgebildet ist, sodass der Knochenzement oder das andere Augmentationsmaterial direkt durch eine Bohrung in der Einbringeinrichtung in die erste Knochenbohrung eingebracht wird und eine zusätzliche, gesonderte Kanüle entfällt.

Es versteht sich, dass alternativ zu Knochenzement auch jedes andere, geeignete Augmentationsmaterial eingesetzt werden kann. Der Begriff Knochenzement im Sinne dieser Anmeldung umfasst daher auch weitere, geeignete Materialien.

Dadurch wird ein Verfahren ermöglicht, wobei die Koppeleinrichtung zweistufig in den Schenkelhals eingebracht wird, nämlich zunächst nur bis zu einer Position, welche beispielsweise 20 mm bis 40 mm, insbesondere etwa 30 mm, von einer Endposition im Schenkelhals entfernt ist, wonach über die kanülierte Einbringeinrichtung Knochenzement in einen Endbereich des Schenkelhals eingebracht wird, üblicherweise mit einer Kanüle, wonach die Koppeleinrichtung bis in die Endposition im Schenkelhals bewegt wird, um den in dieser Endposition befindlichen Knochenzement zu verdrängen, wodurch dieser sich im Knochen verteilt. Dies ist insbesondere bei Patienten mit fortgeschrittener Osteoporose sehr vorteilhaft, um den Knochenzement im porösen Knochen zu verteilen und somit den Knochen zu stabilisieren. Besonders bevorzugt wird der K-Draht auch hier erst dann entfernt, wenn die Koppeleinrichtung vollständig in den Schenkelhals bis zur Endposition eingebracht ist und üblicherweise auch erst dann, wenn der Knochenzement vollständig ausgehärtet ist. Dadurch wird auf zuverlässige Weise verhindert, dass Knochenzement bis zur Gelenkskapsel gelangt, was keinesfalls passieren darf. Dies könnte jedoch passieren, wenn der K-Draht früher entfernt wird, zumal ein Risiko besteht, dass bei einem Einbringen des K-Drahtes in den Oberschenkelkopf der Oberschenkelkopf durchstoßen wird, sodass dadurch eine durchgängige Öffnung bis zur Gelenkskapsel gebildet wird. Anders ausgedrückt verschließt der K-Draht somit eine allfällige Durchgangsöffnung bis in die Gelenkskapsel während des Einbringens des Knochenzementes auf wirkungsvolle Weise, wodurch ein unbeabsichtigtes Eindringen des Knochenzementes in die Gelenkskapsel verhindert ist.

Bevorzugt weist die Koppeleinrichtung endseitig ein Gewinde auf, mit welchem die Koppeleinrichtung im Oberschenkelkopf fixiert werden kann. Für ein Zusammenwirken der Koppeleinrichtung mit der Justiereinrichtung weist die Koppeleinrichtung ferner üblicherweise eine mit der Justiereinrichtung korrespondierende Kontaktfläche auf, welche auch gleichzeitig als Nut ausgebildet sein kann, um eine Rotation mittels der Justiereinrichtung zu verhindern. Die entsprechende Kontaktfläche ist üblicherweise an einer Seite anzuordnen, an welcher die Justiereinrichtung relativ zur Koppeleinrichtung angeordnet ist, üblicherweise distal. Um dennoch eine hohe Variabilität in Bezug auf eine mögliche Einschraubtiefe zu gewährleisten, ist bevorzugt vorgesehen, dass über einen Umfang der Koppeleinrichtung verteilt zumindest zwei entsprechende Kontaktflächen vorgesehen sind. Bei einer Gewindesteigung von beispielsweise 3,2 mm kann die Koppeleinrichtung dann nicht nur in 3,2-mm-Schritten sondern beispielsweise bei zwei über den Umfang gleichmäßig verteilt angeordneten Kontaktflächen in 1,6-mm-Schritten Einschreibtiefe in den Oberschenkelkopf eingebracht werden.

Die Justiereinrichtung weist üblicherweise eine etwa zylindrische oder konische Außenkontur mit einem Gewinde auf, wobei die Justiereinrichtung mittels des Gewindes in den Nagel einschraubbar ist, üblicherweise an einer relativ zur Koppeleinrichtung distal angeordneten Position. Eine Länge der Justiereinrichtung ist üblicherweise größer als eine Länge einer Öffnung im Nagel, durch welche die Justiereinrichtung durch den Nagel geführt wird, sodass die Justiereinrichtung den Nagel üblicherweise beidseits überragt. Es kann auch vorgesehen sein, dass die Justiereinrichtung nahezu so lang wie die Koppeleinrichtung ausgebildet ist, sodass diese von einer lateralen Seite des Nagels bis in einen Bereich des Oberschenkelkopfes ragen kann. Dadurch kann die Koppeleinrichtung stabilisiert und eine Rotation der Koppeleinrichtung um die Querachse sowie eine Rotation eines Oberschenkelkopffragmentes um die Koppeleinrichtung effektiv verhindert werden.

Bei einem Verfahren zur Versorgung einer Schenkelhalsfraktur, wobei ein Nagel in einen intramedullären Kanal eines Oberschenkelknochenschaftes eingebracht wird, wonach in den Nagel eine Koppeleinrichtung eingebracht wird, welche in einem Oberschenkelkopf fixiert wird, wird eine Justiereinrichtung mit dem Nagel verbunden, welche eine Bewegbarkeit der Koppeleinrichtung relativ zum Nagel entlang einer Querachse begrenzt. Üblicherweise wird für ein derartiges Verfahren eine erfindungsgemäße Vorrichtung eingesetzt. Die Querachse steht üblicherweise etwa quer zu einer Längsachse des Nagels, in der Regel unter einem Winkel, unter welchem eine Achse des Oberschenkelhalses zu einer Achse des Oberschenkelknochenschaftes steht, beispielsweise unter einem Winkel von 110° bis 140°.

Günstig ist es, wenn zunächst ein erster K-Draht entlang der Querachse in den Schenkelhals bis in den Oberschenkelkopf eingebracht wird, welcher erste K-Draht einen geringeren Durchmesser als die Koppeleinrichtung aufweist, wonach eine erste Knochenbohrung für die Koppeleinrichtung in den Schenkelhals eingebracht wird, wobei der erste K-Draht als Führung dient, wonach die Koppeleinrichtung durch den Nagel in den Schenkelhals eingebracht wird. Dies gewährleistet eine besonders präzise Einbringung der Koppeleinrichtung in den Schenkelhals, zumal die für einen K-Draht erforderliche Bohrung einen sehr geringen Durchmesser von beispielsweise 1,5 mm aufweist, welche Bohrung mit hoher Präzision eingebracht werden kann. In der Regel wird die Bohrung hierfür mittels des K-Drahtes eingebracht, sodass der K-Draht zunächst als Bohrer und in weiterer Folge als Führung für die größere Knochenbohrung und die Koppeleinrichtung dient.

Üblicherweise dient der K-Draht auch als Führung für das Einbringen der Koppeleinrichtung in den Schenkelhals bzw. den Oberschenkelkopf.

Besonders bevorzugt ist vorgesehen, dass der K-Draht entfernt wird, nachdem die Koppeleinrichtung vollständig in den Schenkelhals eingebracht ist. Der K-Draht wird somit in Lateralrichtung erst nach einem endgültigen Positionieren der Koppeleinrichtung im Schenkelhals entnommen.

Insbesondere bei Frakturen in der Region der Basis des Schenkelhalses kann es vorteilhaft sein, einen zweiten K-Draht achsversetzt in den Oberschenkelkopf einzuführen, um eine Rotation des Oberschenkelkopfes beim Bohren für oder Einschrauben der Koppeleinrichtung zu vermeiden.

Insbesondere bei Osteoporose Patienten kann es vorteilhaft sein, wenn die Koppeleinrichtung zunächst nur bis zu einer Zwischenposition in die erste Knochenbohrung eingeführt wird, wonach Knochenzement in einen Endbereich der ersten Knochenbohrung eingebracht wird, wonach die Koppeleinrichtung vollständig in die erste Knochenbohrung eingebracht wird, wobei ein im Endbereich befindlicher Knochenzement im Oberschenkelkopf verteilt wird. Ein abschließendes Einbringen der Koppeleinrichtung in den Endbereich bewirkt somit ein Verteilen des Knochenzementes, wobei die Koppeleinrichtung als Kolben wirkt, der den Zement verteilt. Die Koppeleinrichtung kann somit in einem ersten Schritt beispielsweise bis etwa 30 mm vor einer finalen Position temporär fixiert werden, wonach der Knochenzement eingebracht wird, wonach die Koppeleinrichtung um weitere 30 mm bis in die finale Endposition in medialer Richtung bewegt wird. Nach einem Aushärten des Knochenzementes wird anschließend der K-Draht entnommen. Sollte bei Einbringen des K-Drahtes in den Oberschenkelkopf der Oberschenkelkopf durchstoßen worden sein, ist somit aufgrund des im Oberschenkelkopf befindlichen K-Drahtes während des Zementierens verhindert, dass Knochenzement durch die gegebenenfalls unbeabsichtigt erzeugte Durchtrittsöffnung vom Oberschenkelkopf in die Gelenkskapsel tritt.

Besonders bevorzugt ist vorgesehen, dass der Knochenzement durch eine kanülierte Einbringeinrichtung in den Endbereich der ersten Knochenbohrung eingebracht wird, welche Einbringeinrichtung durch eine Öffnung, durch welche die Justiereinrichtung durch den Nagel führbar ist, durch den Nagel geführt ist und nach Einbringen des Knochenzementes aus dem Nagel entnommen wird, wonach die Justiereinrichtung in die Öffnung eingeführt, insbesondere eingeschraubt, wird. Die Öffnung kann wie ausgeführt mit der Bohrung zusammenfallen, sodass in einer länglich ausgebildeten Bohrung in einem oberen bzw. proximalen Bereich die Koppeleinrichtung und in einem unteren bzw. distalen Bereich die Justiereinrichtung in der Bohrung angeordnet sind.

Zum Einbringen der Justiereinrichtung, welche ebenfalls bis in den Oberschenkelkopfbereich ragen kann, wird üblicherweise eine zweite Knochenbohrung in den Schenkelhals eingebracht, üblicherweise ebenfalls von einer lateralen Seite des Oberschenkelknochens aus. Hierzu ist bevorzugt vorgesehen, dass distal von einem Bereich, an welchem die Koppeleinrichtung mit dem Nagel verbindbar ist, ein zweiter K-Draht eingebracht wird, insbesondere unter einem Winkel von 0,1° bis 15°, insbesondere 0,8° bis 2°, zum ersten K-Draht, wonach eine zweite Knochenbohrung in den Schenkelhals eingebracht wird, wobei der zweite K-Draht als Führung dient, wonach der zweite K-Draht entfernt wird, wonach in die zweite Knochenbohrung die Justiereinrichtung eingebracht wird, welche den Nagel beidseits überragt.

Weitere Merkmale, Vorteile und Wirkungen der Erfindung ergeben sich anhand der nachfolgend dargestellten Ausführungsbeispiele. In den Zeichnungen, auf welche dabei Bezug genommen wird, zeigen:
Fig. 1 bis 3 eine erfindungsgemäße Vorrichtung in Schnittdarstellung und unterschiedlichen Arbeitszuständen;
Fig. 4 bis 6 Details weiterer erfindungsgemäßer Vorrichtungen;
Fig. 7 bis 14 unterschiedliche Prozessschritte bei Durchführung eines erfindungsgemäßen Verfahrens;
Fig. 15 eine weitere erfindungsgemäße Vorrichtung;
Fig. 16 bis 26 unterschiedliche Prozessschritte bei einem weiteren erfindungsgemäßen Verfahren;
Fig. 27 bis 29 ein Detail einer erfindungsgemäßen Vorrichtung in schematischer Darstellung;
Fig. 30 und 31 Details einer weiteren erfindungsgemäßen Vorrichtung.

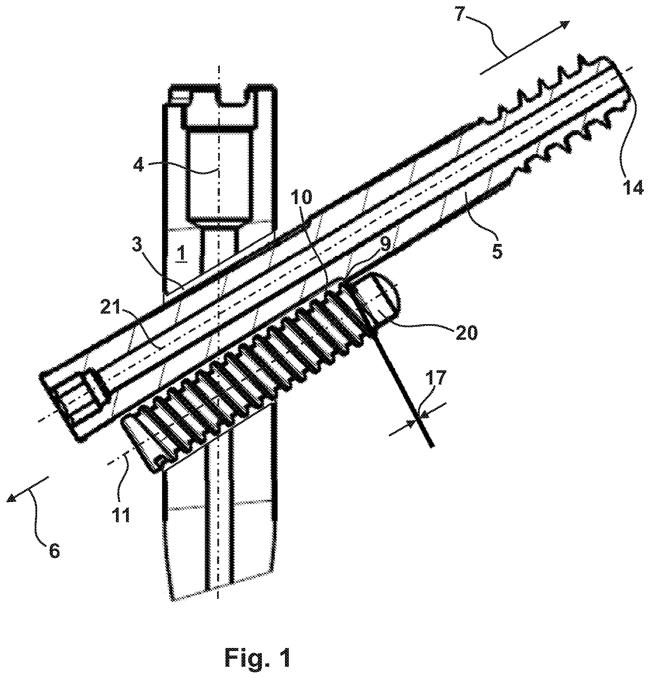

Fig. 1 bis 3 zeigen eine erfindungsgemäße Vorrichtung in unterschiedlichen Arbeitszuständen. Die Vorrichtung weist einen nur teilweise dargestellten Knochennagel auf, welcher entlang einer Längsachse 4 in einem intramedullären Kanal eines Oberschenkelknochens angeordnet werden kann, welcher intramedulläre Kanal an einem oberen bzw. proximalen Ende des Oberschenkelknochens beginnt. Weiter weist die Vorrichtung eine hier als Knochenschraube 5 ausgebildete Koppeleinrichtung auf, welche in einer Bohrung 3 des Nagels 1 angeordnet ist, um den Oberschenkelknochenschaft 2 mit einem abgebrochenen Oberschenkelkopf 8 zu verbinden. Hierzu ist an einem medialen Ende 14 der Knochenschraube 5 ein Gewinde vorgesehen, welches in den Oberschenkelkopf 8 eingeschraubt werden kann. Die Bohrung 3 und die Knochenschraube 5 sind dabei derart ausgerichtet, dass die Knochenschraube 5 entlang einer Querachse 21, welche quer zur Längsachse 4 des Nagels 1, üblicherweise unter einem Winkel von etwa 130° zur Längsachse 4 des Nagels 1, ausgerichtet ist, mit dem Nagel 1 verbunden werden kann, sodass sich die Querachse 21 etwa entlang eines Oberschenkelhalses und der Nagel 1 etwa entlang des Oberschenkelknochenschaftes 2erstreckt.

Mit dem Nagel 1 ist die Knochenschraube 5 nicht verschraubt, sondern entlang einer Querachse 21 bewegbar verbunden, wodurch eine besonders gute Heilung erreicht wird. Bei der erfindungsgemäßen Vorrichtung ist ferner eine Justiereinrichtung vorgesehen, welche hier durch eine Justierschraube 20 gebildet ist, welche mit dem Nagel 1 verschraubbar ist, und zwar in einer Öffnung, welche hier in einem unteren Bereich jener Bohrung 3 angeordnet ist, durch welche die Knochenschraube 5 den Nagel 1 durchsetzt.

Die Justierschraube 20 begrenzt hierbei eine Bewegbarkeit der Knochenschraube 5 relativ zum Nagel 1, abhängig von einer Einschraubtiefe. Hierzu ist einerseits eine erste Anschlagfläche 9 vorgesehen, welche eine Bewegbarkeit der Knochenschraube 5 in Lateralrichtung 6 begrenzt. Die erste Anschlagfläche 9 an der Justierschraube 20 bildet somit eine laterale Endposition der Knochenschraube 5 und ist etwa normal zu einer Achse der Justierschraube 20 bzw. einer Justierrichtung 11 orientiert. Beispielsweise kann die erste Anschlagfläche 9 an der Justierschraube 20 durch eine Gewindeflanke gebildet sein.

Ferner ist an der Justierschraube 20 auch eine zweite Anschlagfläche 10 vorgesehen, welche eine Bewegbarkeit der Knochenschraube 5 in Medialrichtung 7 begrenzt. Die zweite Anschlagfläche 10 ist an einer Umfangsfläche der Justierschraube 20 angeordnet und somit etwa normal zur ersten Anschlagfläche 9 und etwa koaxial zur Justierrichtung 11 orientiert. Diese zweite Anschlagfläche 10 ergibt sich durch einen Winkel α zwischen Justierrichtung 11 und Querrichtung von etwa 1,2°.

In Fig. 1 ist eine Position der Justierschraube 20 dargestellt, in welcher diese Justierschraube 20 gänzlich in den Nagel 1 eingeschraubt ist, sodass die Knochenschraube 5 nicht relativ zum Nagel 1 bewegbar ist, sondern die Knochenschraube 5 sowohl an der ersten Anschlagfläche 9 als auch an der zweiten Anschlagfläche 10 an der Justierschraube 20 anliegt.

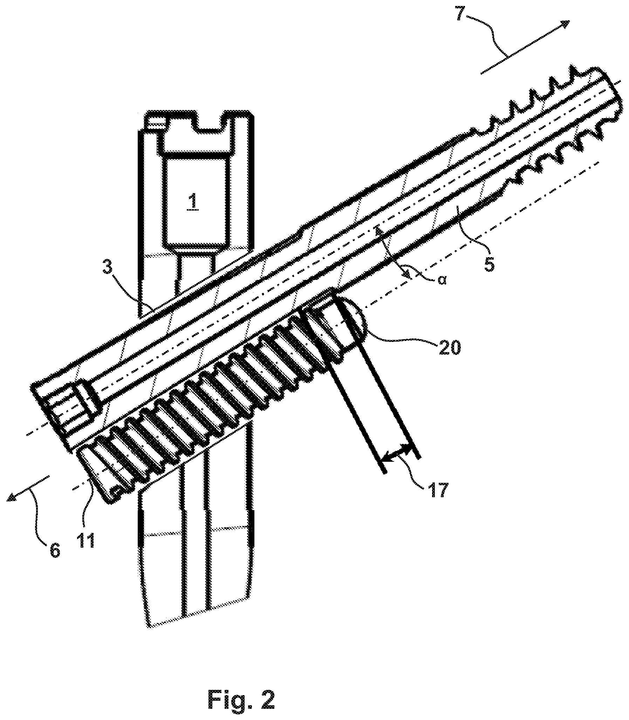

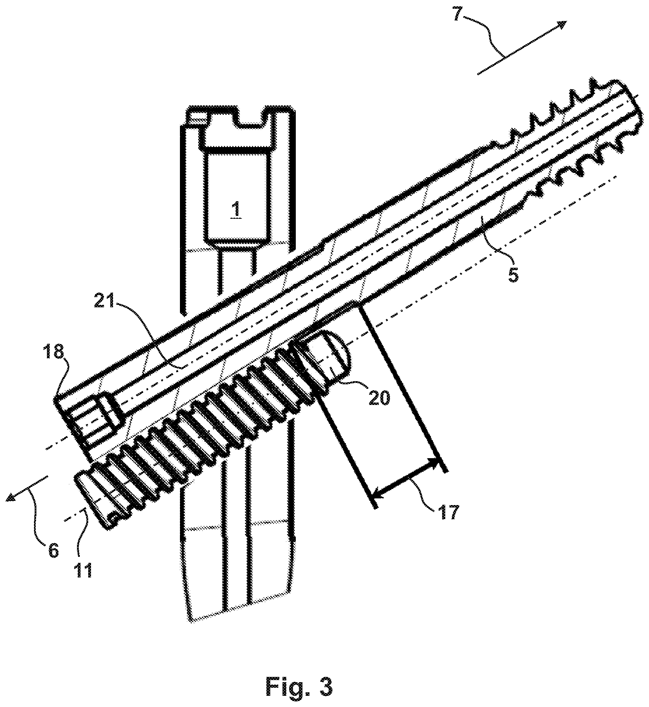

Fig. 2 und 3 zeigen weitere Arbeitspositionen, wobei die Justierschraube 20 in Fig. 2 gegenüber Fig. 1 etwas in Lateralrichtung 6 aus dem Nagel 1 geschraubt ist, wodurch sich eine Bewegbarkeit bzw. ein Spiel 17 der Knochenschraube 5 relativ zur Justierschraube 20 bzw. relativ zum Nagel 1 in Lateralrichtung 6 bzw. entgegengesetzt in Medialrichtung 7 ergibt.

Fig. 3 zeigt eine weitere Arbeitsposition, wobei die Justierschraube 20 gegenüber Fig. 2 noch weiter in Lateralrichtung 6 aus dem Nagel 1 geschraubt ist, sodass das Spiel 17 der Knochenschraube 5 größer ist als in der in Fig. 2 dargestellten Arbeitsposition.

Eine Bewegbarkeit der Knochenschraube 5 relativ zum Knochennagel ist dabei durch die Justierschraube 20 in Lateralrichtung 6 durch eine erste Anschlagfläche 9 begrenzt, welche hier an einem medialen Ende 14 der Justierschraube 20 angeordnet ist. Eine Bewegbarkeit in Richtung der Querachse 21 in Medialrichtung 7 ist durch eine zweite Anschlagfläche 10 an der Justierschraube 20 begrenzt, welche durch eine etwa zylindrische Außenfläche der Justierschraube 20 gebildet ist. Dies ergibt sich durch einen Winkel α, unter welchem eine Justierachse der Justierschraube 20 zur Querachse 21 orientiert ist, sodass eine Bewegung der Justierschraube 20 entlang der Justierrichtung 11 unterschiedliche Auswirkungen auf Lagen von erster Kontaktfläche und zweiter Kontaktfläche hat. Dadurch verschiebt sich mit einem Bewegen der Justierschraube 20 entlang der Justierrichtung 11 nicht nur eine Lage der Anschlagflächen, sondern ändert sich auch ein Spiel 17 der Knochenschraube 5 relativ zum Nagel 1 und relativ zur Justierschraube 20. Dabei können der Winkel und Abmessungen von Knochenschraube und Justierschraube auch derart gewählt sein, dass eine Lage der zweiten Anschlagfläche bzw. eine mediale Endposition der Knochenschraube relativ zum Nagel nicht durch eine Änderung der Position der Justierschraube verändert wird, sondern sich eine Änderung der Einschraubtiefe nur auf die Position der ersten Anschlagfläche auswirkt.

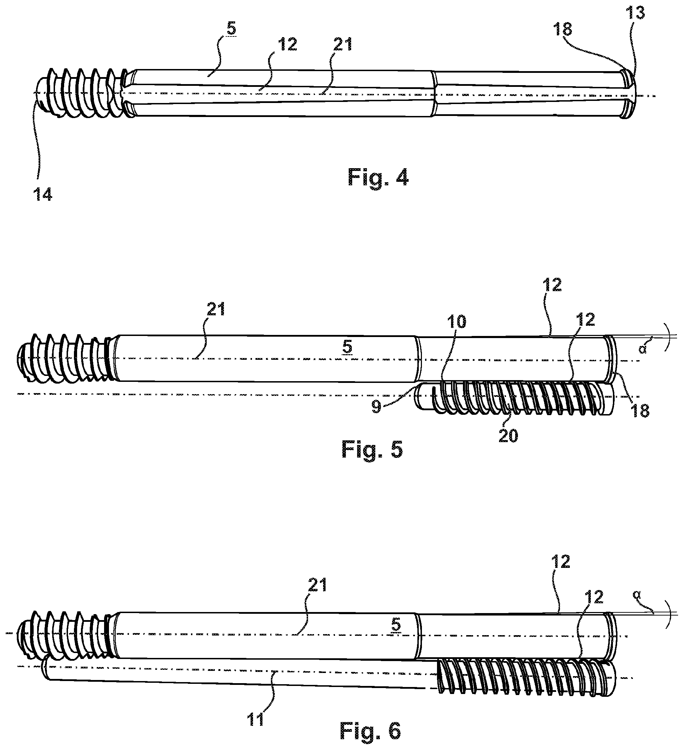

Fig. 4 bis 6 zeigen weitere Ausführungsbeispiele von Koppeleinrichtung und Justiereinrichtung erfindungsgemäßer Vorrichtungen. Auch hier ist die Koppeleinrichtung jeweils als Knochenschraube 5 mit einem medialen Gewinde und ist die Justiereinrichtung jeweils als Justierschraube 20 ausgebildet. Insbesondere in Fig. 4 ist auch eine mit erster Kontaktfläche und zweiter Kontaktfläche der Justierschraube 20 korrespondierende Nut 12 entlang einer Längsrichtung an der Knochenschraube 5 ersichtlich, welche einerseits zu erster Kontaktfläche und zweiter Kontaktfläche korrespondierende Kontaktflächen an der Knochenschraube 5 bereitstellt und andererseits eine Rotation der Knochenschraube 5 um die Querachse 21 verhindert, wenn die Justierschraube 20 im Bereich dieser Kontaktfläche angeordnet ist. Die Nut 12 korrespondiert hierzu mit der Justierschraube 20 und ist vorzugsweise in der Arbeitsposition etwa parallel zur Justierrichtung 11. Folglich steht die Nut 12 üblicherweise unter einem Winkel α zur Querachse 21 von beispielsweise 1,2°.

Die entsprechende Nut 12, welche üblicherweise zur ersten Anschlagfläche 9 und zur zweiten Anschlagfläche 10 korrespondierende Flächen aufweist, kann nur einmal an der Knochenschraube 5 vorgesehen sein, üblicherweise etwa entlang einer Achse der Knochenschraube 5 bzw. der Querachse 21. Bevorzugt ist jedoch vorgesehen, dass diese Nut 12 über einen Umfang der Knochenschraube 5 verteilt mehrmals, zumindest zweimal, wie in Fig. 5 und Fig. 6 dargestellt angeordnet ist, zumal dann mehrere Möglichkeiten bestehen, wie die Knochenschraube 5 in den Nagel 1 eingeschraubt werden kann. So ist beispielsweise bei einer Gewindesteigung von 3,2 mm eine Einschraubtiefe in 1,6 mm Schritten möglich, wenn die entsprechende Nut 12 an zwei gegenüberliegenden Positionen über den Umfang verteilt angeordnet ist, zumal eine der Nuten 12 in der Arbeitsposition mit Vorteil an einer mit der Justierschraube 20 korrespondierenden Position angeordnet ist.

Wie in Fig. 5 und Fig. 6 ersichtlich, stehen die Querachse 21 und die Justierachse unter einem Winkel α zueinander, wodurch sich eine Rampe ergibt, die zu der unterschiedlichen Auswirkung der Bewegung der Justierschraube 20 in Justierrichtung 11 auf die einzelnen erste Anschlagfläche 9 und die zweite Anschlagfläche 10 führt.

Wie in Fig. 5 und Fig. 6 ersichtlich, kann die Justierschraube 20 auch als lange Schraube ausgebildet sein, welche bis in einen Bereich des Oberschenkelkopfes 8 ragt, um eine Rotation des Oberschenkelkopfes 8 um die Knochenschraube 5 bei Belastung effektiv zu verhindern.

Wie in den Ausführungsbeispielen der Fig. 4 bis 6 ersichtlich weisen die Knochenschrauben 5 gemäß diesen Ausführungen jeweils an einem lateralen Ende 13 einen Kragen 18 auf, welcher als Anschlag dienen und in Zusammenwirken mit der Justierschraube 20 und/oder dem Nagel 1 eine mediale Endposition der Knochenschraube 5 definieren kann. Allerdings kann dieser Kragen 18 auch entfallen, zumal eine mediale Migration auch durch ein Zusammenwirken von Knochenschraube 5 und Justierschraube 20 definiert sein kann.

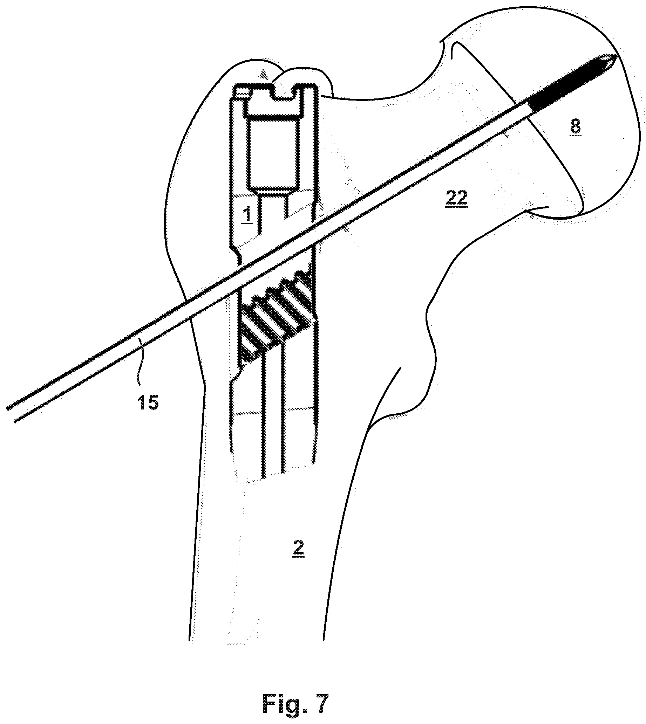

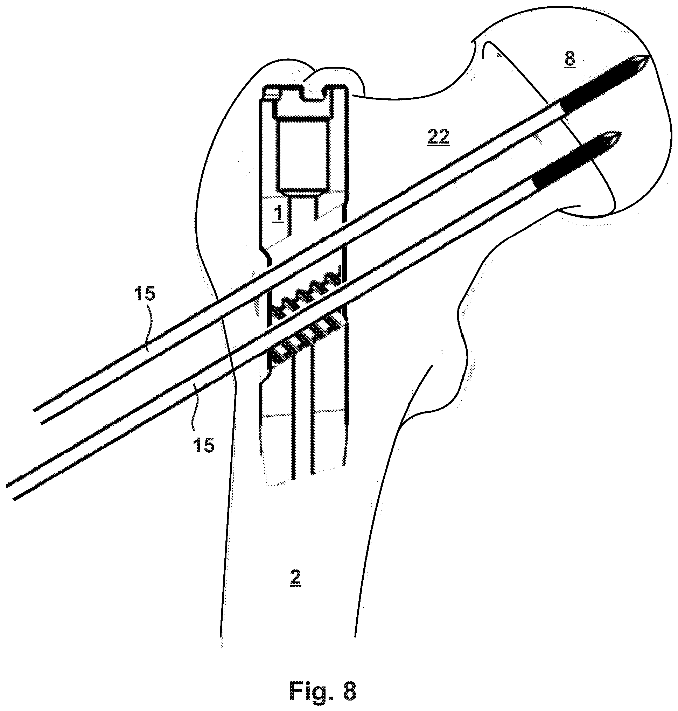

Fig. 7 bis 14 zeigen verschiedene Schritte eines erfindungsgemäßen Verfahrens zum Versorgen einer Oberschenkelfraktur. Wie in Fig. 7 und Fig. 8 ersichtlich werden zunächst mit einem Bohrer mit geringem Durchmesser K-Draht-Bohrungen in den Schenkelhals 22 eingebracht, in welchen anschließend K-Drähte 15 positioniert werden. Üblicherweise sind hierzu die K-Drähte selbst als Bohrer ausgebildet, sodass mit diesen die K-Draht-Bohrungen eingebracht werden und die K-Drähte 15 anschließend im Knochen verbleiben. Anschließend werden die K-Drähte 15 genutzt, um Bohrer mit größeren Durchmessern zu führen, sodass eine erste Knochenbohrung 23 für die auch hier als Knochenschraube 5 ausgebildete Koppeleinrichtung und eine zweite Knochenbohrung 24 für die auch hier als Justierschraube 20 ausgebildete Justiereinrichtung präzise eingebracht werden können.

Fig. 7 zeigt dabei eine Situation, in welcher ein erster K-Draht 15 eingebracht wurde.

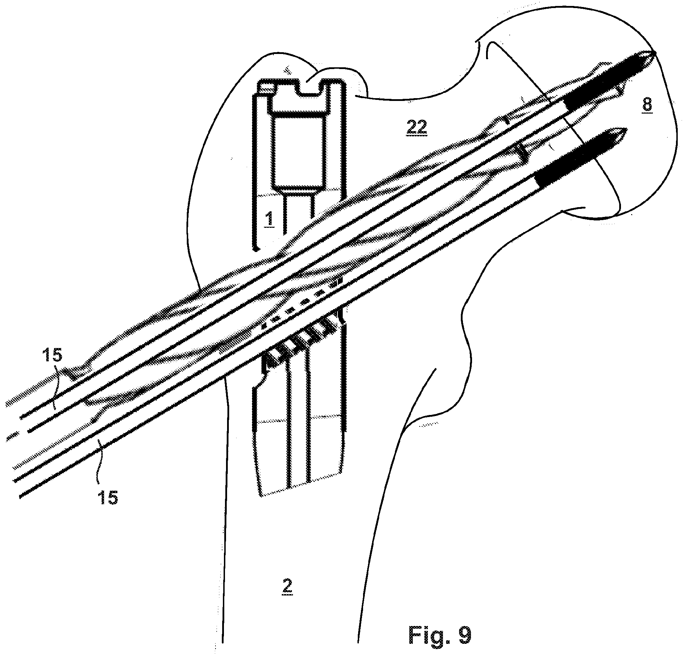

Fig. 8 zeigt einen Verfahrensschritt, in welchem der zweite K-Draht 15 eingebracht wurde. Fig. 9 zeigt einen Verfahrensschritt, in welchem eine erste Knochenbohrung 23 für die Knochenschraube 5 in den Schenkelhals 22 eingebracht wird, wobei ein Bohrer hohl ausgebildet ist und durch den entsprechenden K-Draht 15 geführt wird. Hierzu kann auch eine zusätzliche Positionierhilfe vorgesehen sein, beispielsweise mittels Röntgen oder dergleichen.

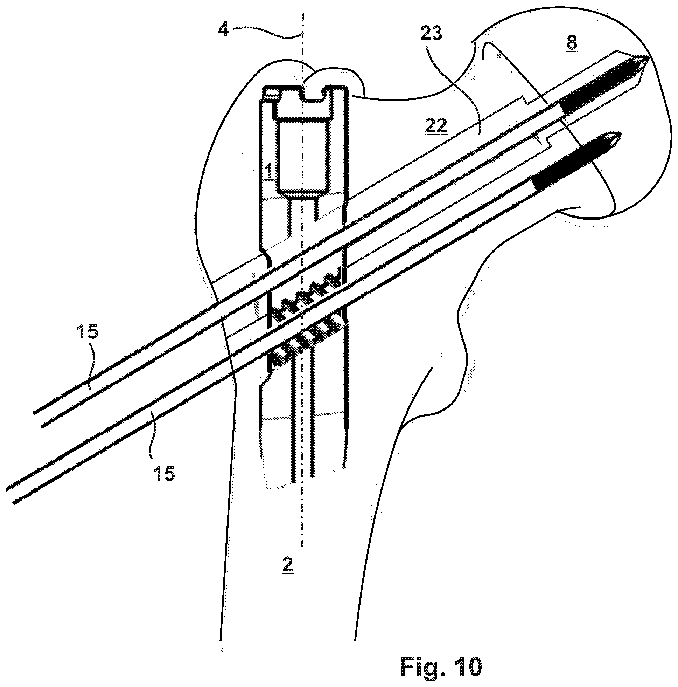

Fig. 10 zeigt eine Situation, in welcher die erste Knochenbohrung 23 bereits gebildet ist, jedoch die zweite Knochenbohrung 24 noch nicht gebildet ist. Wie ersichtlich bleibt der K-Draht 15 auch nach dem Bohren der ersten Knochenbohrung 23 in der ersten Knochenbohrung 23.

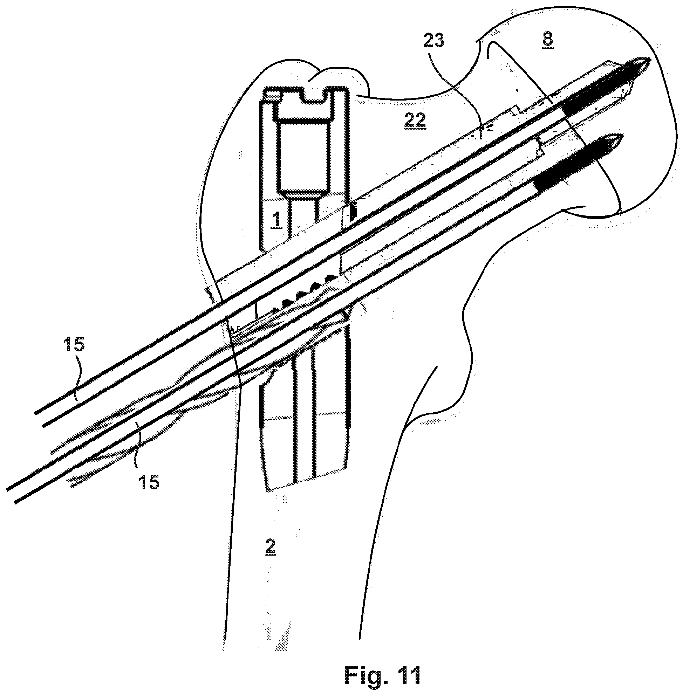

Fig. 11 zeigt einen Verfahrensschritt, in welchem die zweite Knochenbohrung 24 gebohrt wird, wieder unter Nutzung eines K-Drahtes 15 als Führung.

Alternativ zum Einsatz eines K-Drahtes 15 als Führung kann die zweite Knochenbohrung 24 auch ohne K-Draht 15, also ohne Führung, und mit einem soliden Bohrer eingebracht werden.

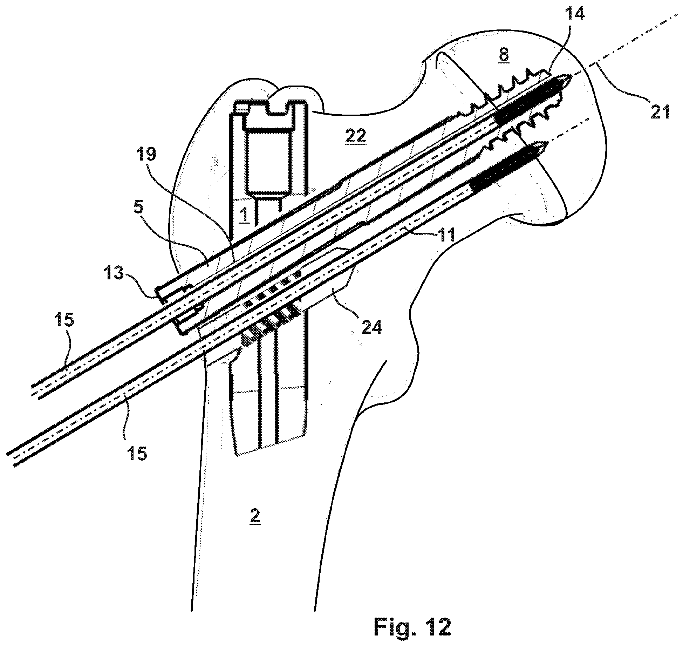

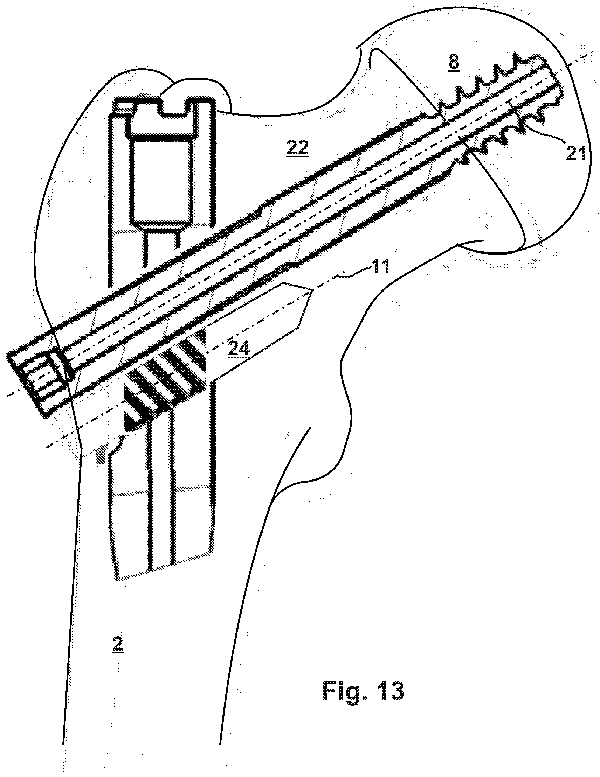

Fig. 12 zeigt einen Verfahrensschritt, wobei auch die zweite Knochenbohrung 24 bereits fertiggestellt ist und die Knochenschraube 5 in die erste Knochenbohrung 23 eingebracht wird, und zwar ebenfalls mittels des K-Drahtes 15, indem der K-Draht 15 als Führung für die Knochenschraube 5 dient. Die Knochenschraube 5 weist hierzu eine mittige Durchgangsbohrung 19 auf, welche sich von einem medialen Ende 14 bis zu einem lateralen Ende 13 erstreckt. Der K-Draht 15 wird daher erst entfernt, wenn die Knochenschraube 5 vollständig in die erste Knochenbohrung 23 eingebracht wurde. Dann wird auch der zweite K-Draht 15 entfernt wie in Fig. 13 ersichtlich.

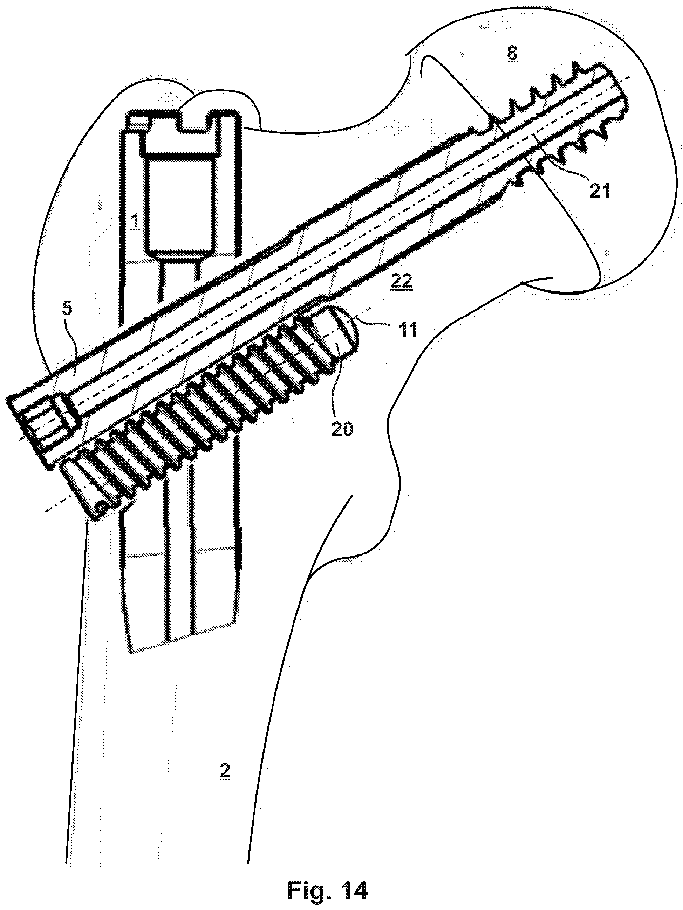

Schließlich wird die Justierschraube 20 eingebracht wie in Fig. 14 ersichtlich. Durch eine Position der Justierschraube 20 entlang der Justierrichtung 11, welche mittels des Gewindes an der Justierschraube 20 und dem Nagel 1 veränderbar ist, kann eine Bewegbarkeit der Knochenschraube 5 relativ zum Nagel 1 beeinflusst werden.

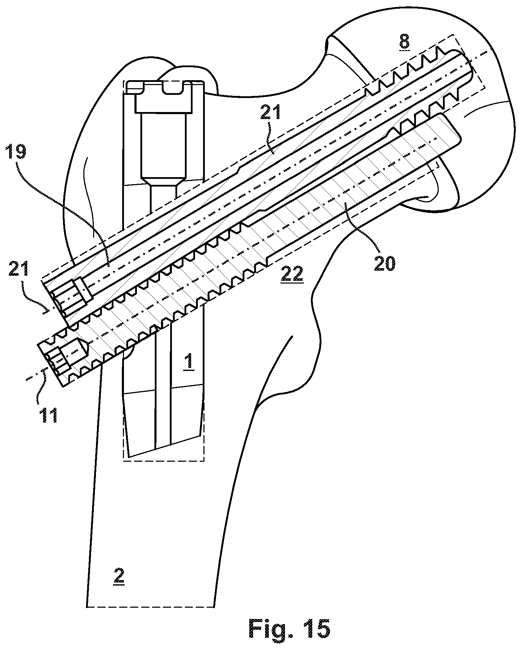

Fig. 15 zeigt eine alternative Ausführungsform, wobei eine lange Justierschraube 20 vorgesehen ist, welche bis in einen Bereich des Oberschenkelkopfes 8 ragt. Bei einer derartigen Ausbildung ist eine Rotation des Oberschenkelkopfes 8 um die Knochenschraube 5 auf effektive Weise verhindert. Die Justierschraube 20 dient somit auch als Rotationssicherung des Oberschenkelkopfes 8 gegenüber dem Oberschenkelknochenschaft 2.

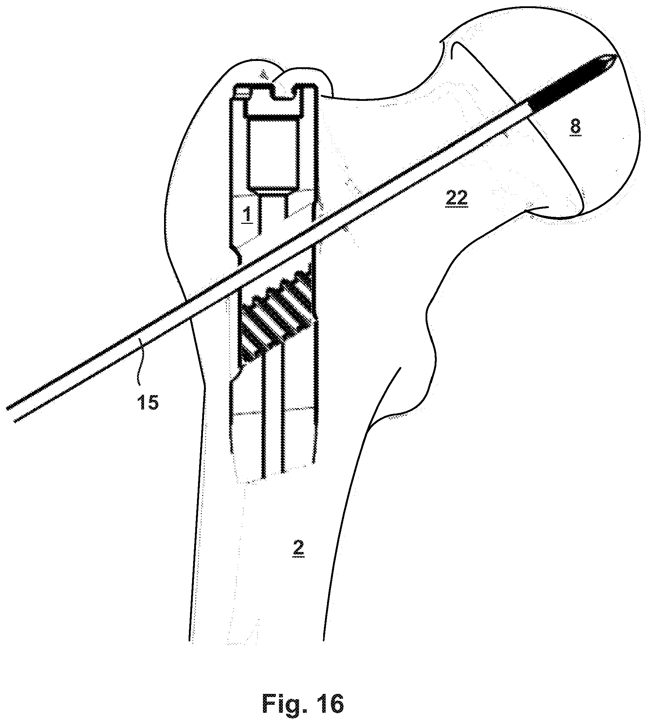

Fig. 16 bis 26 zeigen ein weiteres erfindungsgemäßes Verfahren zur Versorgung einer Oberschenkelfraktur, wobei auch ein Knochenzement 26 in den Oberschenkelkopf 8 eingebracht wird.

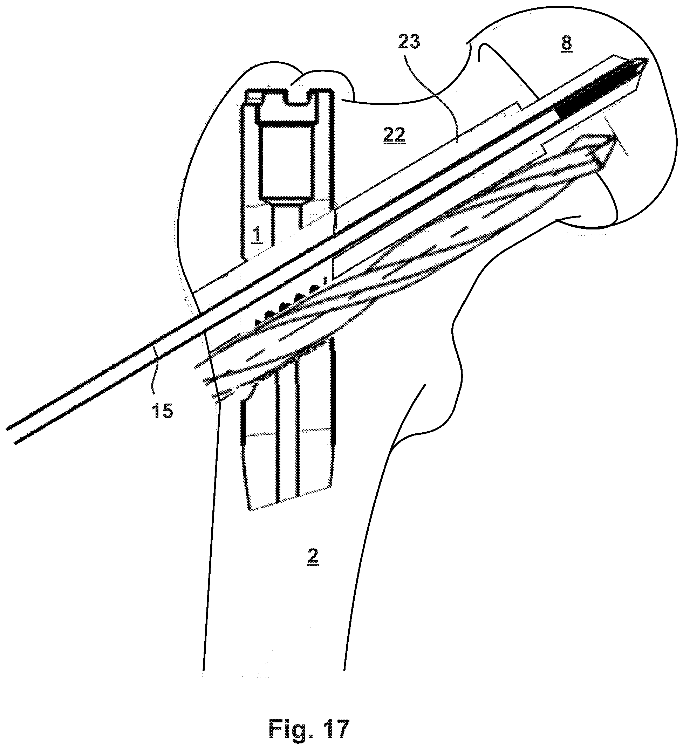

Wie in Fig. 16 und 17 dargestellt, wird hierzu zunächst wieder eine erste Knochenbohrung 23 in den Schenkelhals 22 eingebracht, wobei auch hier wieder zuvor ein K-Draht 15 eingebracht wird, welcher anschließend als Führung für den größeren Bohrer dient.

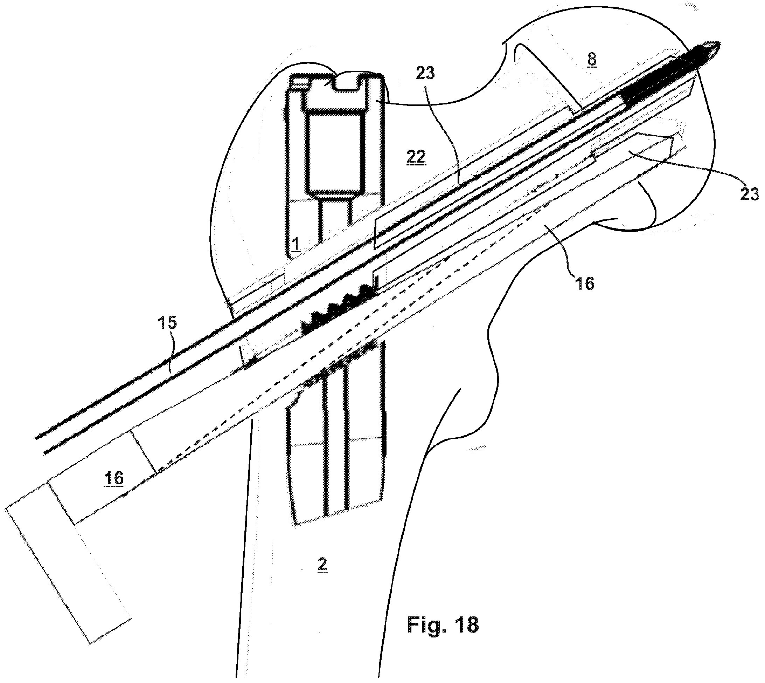

Das hier dargestellte Verfahren betrifft die Versorgung einer Oberschenkelfraktur bei einem Osteoporose-Patienten, weswegen vor einem finalen Einbringen der Knochenschraube 5 und der Justierschraube 20 in einen Endbereich des Oberschenkelkopfes 8 Knochenzement 26 eingebracht wird. Hierzu ist eine Einbringeinrichtung 16 vorgesehen, welche in Fig. 18 bis 23 dargestellt ist. Die Einbringeinrichtung 16 wird durch eine Öffnung in den Nagel 1 und in die zweite Knochenbohrung 24 in den Schenkelhals 22 eingebracht, durch welche Öffnung alternativ und auch in weiterer Folge die Justierschraube 20 durch den Nagel 1 eingebracht wird. Diese Öffnung ist auch hier Teil der Bohrung 3, durch welche die Knochenschraube 5 durch den Nagel 1 geführt ist.

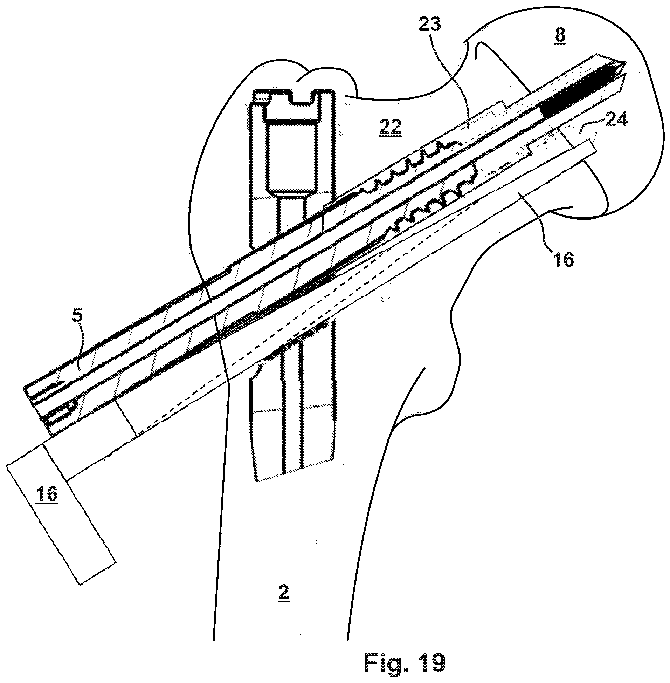

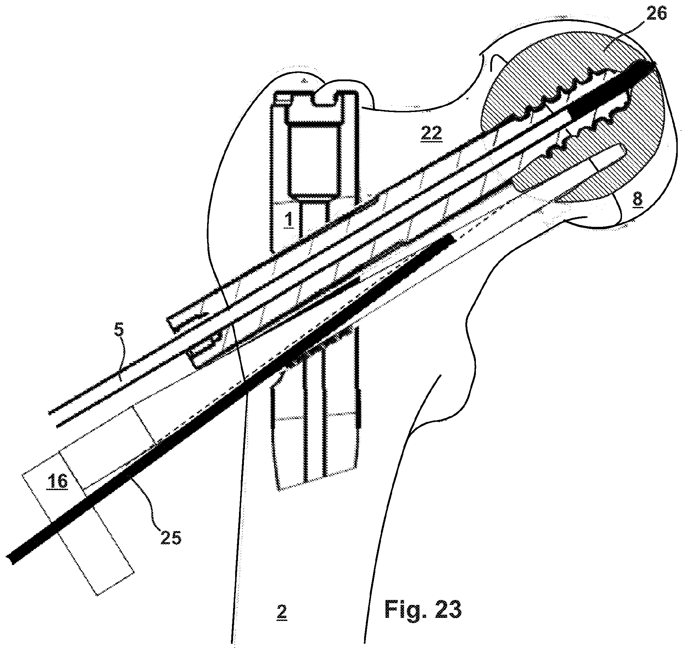

Die dargestellte Einbringeinrichtung 16 weist somit zumindest in einem Bereich, in welchem diese in Kontakt mit dem Nagel 1 steht, eine mit der Justierschraube 20 korrespondierende Außenkontur, jedoch kein Gewinde, auf, wenngleich dies natürlich möglich wäre. Die Einbringeinrichtung 16 ist ferner kanüliert, sodass nach einem Positionieren derselben unter der Knochenschraube 5 durch die Kanülierung mittels einer Kanüle 25 Knochenzement 26 in den Endbereich der ersten Knochenbohrung 23 eingebracht werden kann. Hierzu wird die Knochenschraube 5 zunächst nicht vollständig in die erste Knochenbohrung 23 eingebracht, sondern wie in Fig. 19 bis 22 ersichtlich nur bis zu einer Einbringposition eingebracht, welche Einbringposition beispielsweise 30 mm von einer Endposition entfernt ist, in welcher sich die Knochenschraube 5 erst im in Fig. 23 dargestellten Verfahrensschritt befindet.

Während die Knochenschraube 5 an dieser in Fig. 19 bis 22 dargestellten Einbringposition gehalten wird, wird Knochenzement 26 mittels einer Kanüle 25, die in die Einbringeinrichtung 16 eingeführt wird, in den Endbereich der ersten Knochenbohrung 23 bzw. in einen Bereich des Oberschenkelkopfes 8 eingebracht.

Alternativ zu einer Kanüle 25, die in die Einbringeinrichtung 16 temporär eingeführt wird, kann die Einbringeinrichtung 16 auch selbst als Kanüle 25 ausgebildet sein.

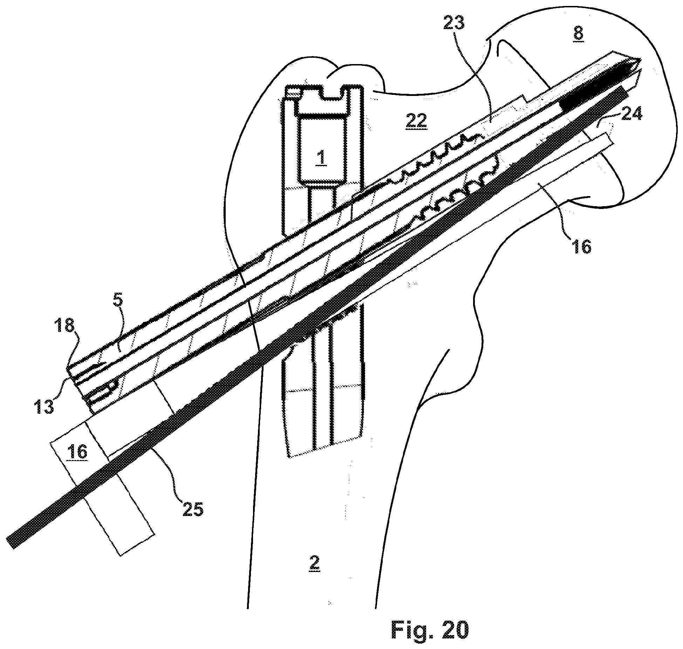

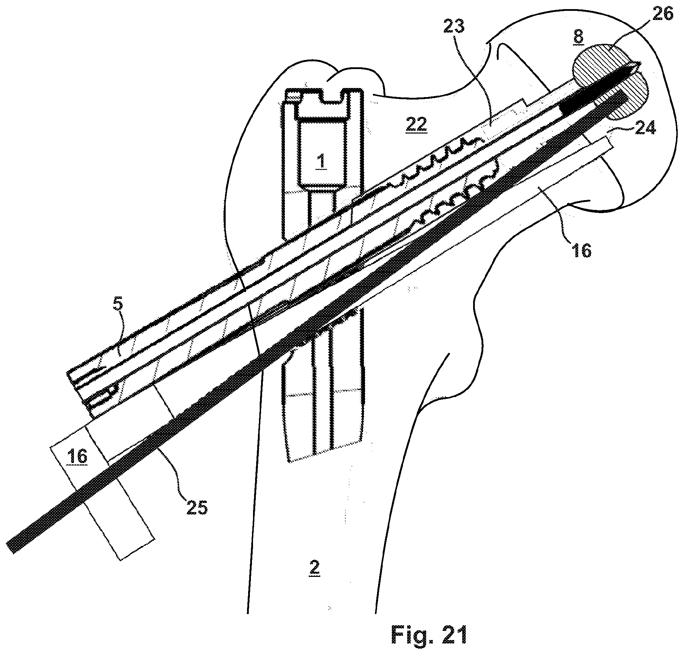

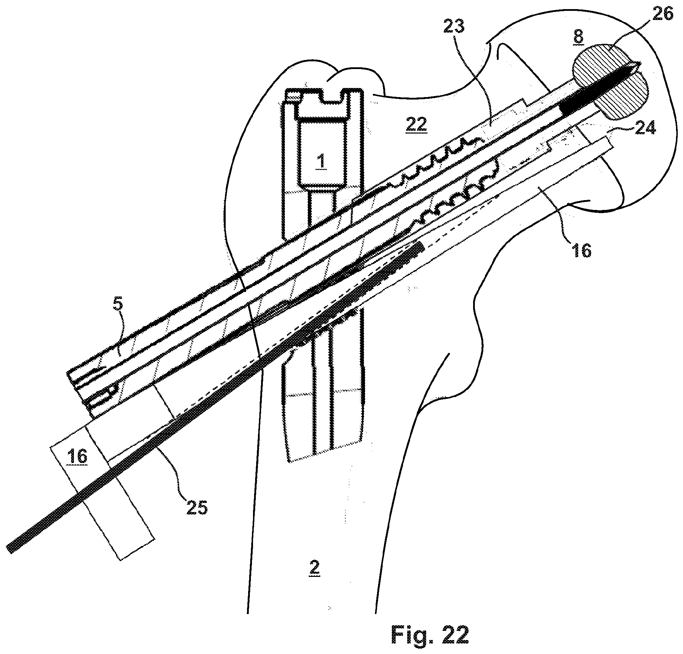

Ein Einbringen der Kanüle 25 in die Kanülierung der Einbringeinrichtung 16, welche als Bohrung ausgebildet ist, ist in Fig. 20 und das Einbringen des Knochenzementes 26 durch die Kanüle 25 in den Oberschenkelkopf 8 ist in Fig. 21 dargestellt. Fig. 22 zeigt die Entfernung der Kanüle 25 aus der Einbringeinrichtung 16, während die Einbringeinrichtung 16 in der zweiten Knochenbohrung 24 verbleibt.

In weiterer Folge wird die Knochenschraube 5 vollständig in die Endposition in der ersten Knochenbohrung 23 eingeschraubt, und zwar vor einem Aushärten des Knochenzementes 26, wobei der in der Endposition befindliche, nicht ausgehärtete Knochenzement 26 im Oberschenkelkopf 8 verteilt wird wie in Fig. 23 dargestellt.

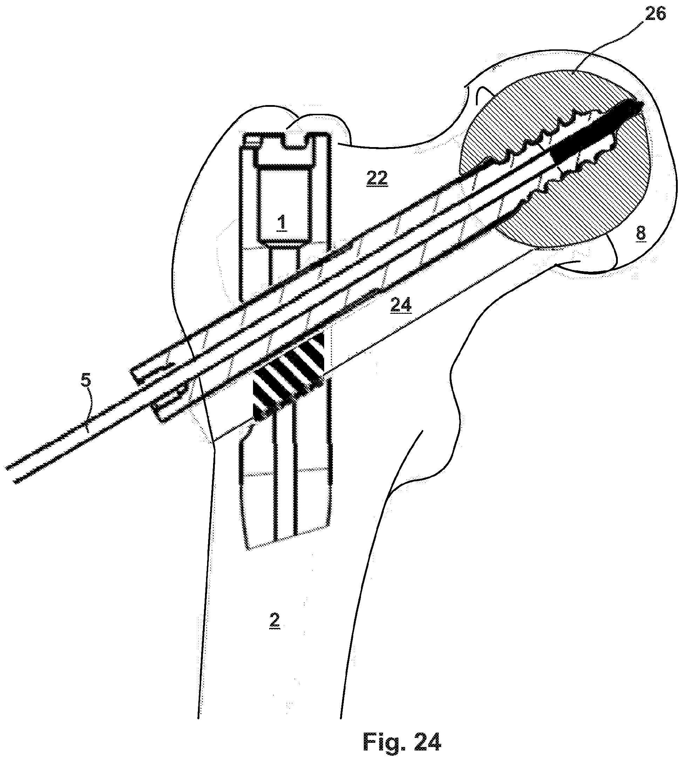

Anschließend wird die Einbringeinrichtung 16 aus der zweiten Knochenbohrung 24 und der Bohrung 3 des Nagels 1 entnommen, wie in Fig. 24 ersichtlich.

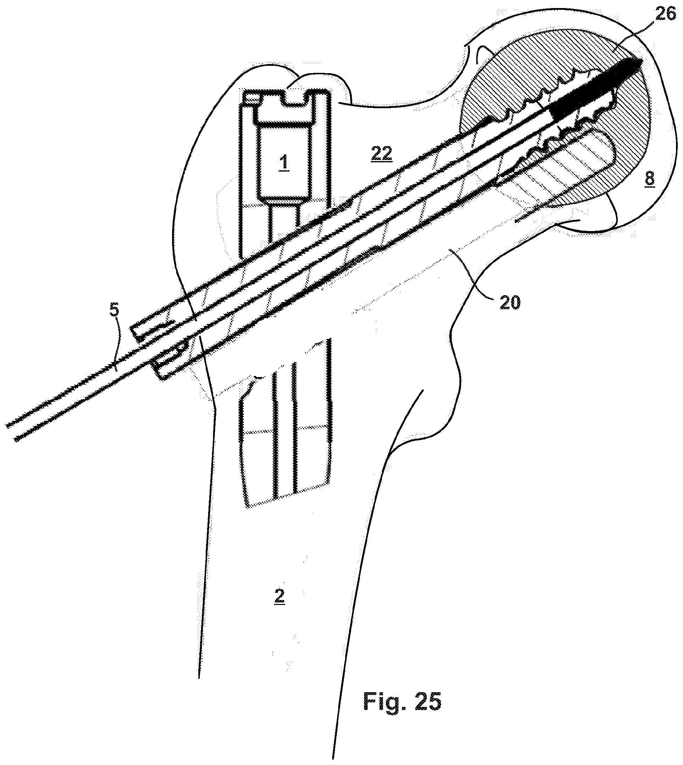

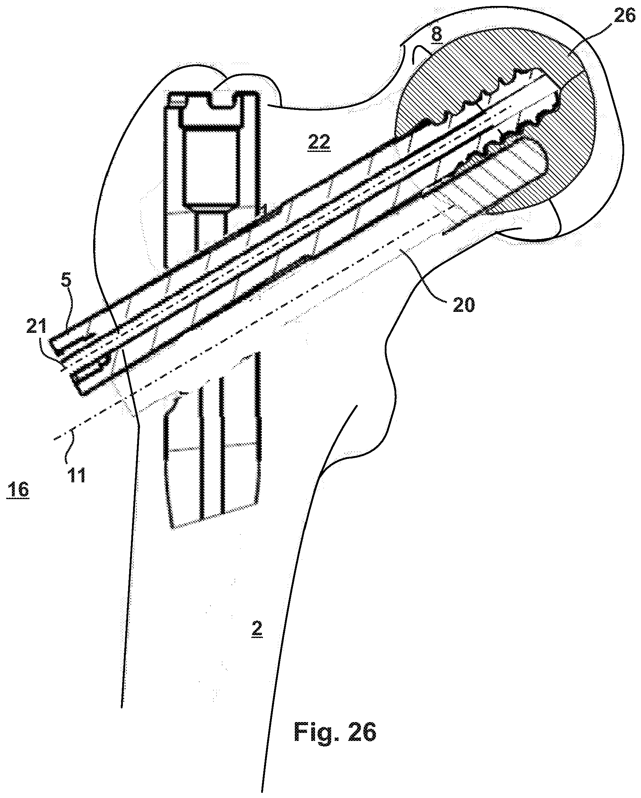

Wie in Fig. 25 ersichtlich wird in weiterer Folge die Justierschraube 20 distal bzw. unterhalb von der Knochenschraube 5 in den Nagel 1 eingeschraubt und schließlich, nach Aushärten des Knochenzementes 26, der K-Draht 15 aus der Knochenschraube 5 entnommen, welcher die Knochenschraube 5 bei einem Einbringen führt. Diese Position, welche einen Abschluss des Einbringens der Vorrichtung zur Versorgung der Fraktur bildet, ist in Fig. 26 dargestellt. Die dargestellte Justierschraube 20 reicht bis in den Bereich des Oberschenkelkopfes 8, sodass diese nicht nur eine Einstellbarkeit des Spiels 17 der Knochenschraube 5 erlaubt und eine Rotation der Knochenschraube 5 um die Querachse 21 verhindert, sondern auch eine Rotation des Oberschenkelkopfes 8 um die Querachse 21 und somit ein Lösen einer Verschraubung zwischen Oberschenkelkopf 8 und Knochenschraube 5 blockiert.

Durch ein Entfernen des K-Drahtes 15 aus der ersten Knochenbohrung 23 erst nach Aushärtung des Knochenzementes 26 ist effektiv verhindert, dass bei einem unbeabsichtigten Durchstoßen des Oberschenkelkopfes 8 mit dem K-Draht 15 bei einem Einbringen desselben der flüssige Knochenzement 26 vor einem Aushärten in die Gelenkskapsel eindringt, zumal der K-Draht 15 eine allfällige, unbeabsichtigt verursachte Öffnung im Oberschenkelkopf 8 verschließt.

Auch bei dieser Ausführung kann an einem lateralen Ende 13 der Knochenschraube 5 ein Kragen 18 vorgesehen sein, um eine durch einen Formschluss mit dem Nagel 1 und/oder der Justierschraube 20 bzw. einen Anschlag eine mediale Endposition zu definieren.

Eine erfindungsgemäße Vorrichtung ermöglicht eine besonders wirkungsvolle Versorgung einer Oberschenkelfraktur, zumal die Bewegbarkeit der Knochenschraube 5 relativ zum Nagel 1 und somit eine Bewegbarkeit des Oberschenkelkopfes 8 relativ zum Oberschenkelknochenschaft auch noch während einer Operation einfach verändert werden kann, um die Bewegbarkeit besonders gut an den jeweiligen Patienten anpassen zu können.

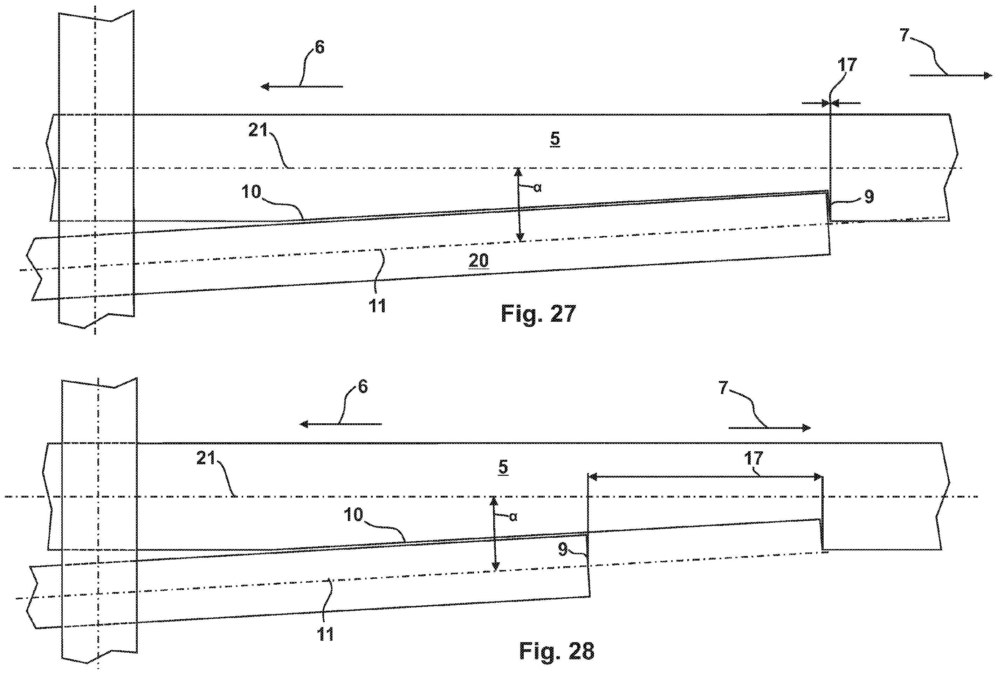

Fig. 27 bis 29 zeigen schematisch ein Zusammenwirken von Knochenschraube 5 und Justierschraube 20 bei einer Ausführungsvariante einer erfindungsgemäßen Vorrichtung ähnlich den in Fig. 1 bis 3 dargestellten Situationen, jedoch hier mit einer Detaildarstellung des Zusammenwirkens von erster Kontaktfläche, zweiter Kontaktfläche und Nut 12. Weiter ist hier, um das Zusammenwirken besonders gut zu veranschaulichen, eine Variante mit einem Winkel α von etwa 3°, einer sich nahezu bis zur Querachse 21 erstreckenden Nut 12 und einem Nagel 1 mit im Vergleich zu Knochenschraube 5 und Justierschraube 20 sehr geringem Durchmesser dargestellt, während der Winkel α bei realen Vorrichtungen üblicherweise weniger als 2° beträgt, die Nut 12 auch weniger stark ausgeprägt sein kann und Durchmesser von Nagel 1, Knochenschraube 5 und Justierschraube 20 auch von den in Fig. 27 bis Fig. 29 dargestellten abweichen können, wie insbesondere in Fig. 1 bis 26 dargestellt.

Wie ersichtlich korrespondiert die Nut 12 in der Knochenschraube 5 mit der Justierschraube 20, und zwar sowohl in Bezug auf Abmessungen als auch in Bezug auf einen Winkel α, unter welchem die Nut 12 und die Justierschraube 20 zur Querachse 21 stehen.

Wenn, wie hier, die Justierschraube 20 durch einen im Bereich der Nut 12 im Wesentlichen zylindrischen Körper gebildet wird und die Nut 12 entsprechend ausgebildet ist, bildet eine Zylindermantelfläche der Justierschraube 20, welche rotationssymmetrisch zur Justierachse ist, in Zusammenwirken mit einer entsprechenden Fläche der Nut 12 eine zweite Anschlagfläche 10, und zwar bereits ab einer Einschraubtiefe der Justierschraube 20 in den Nagel 1, ab welcher Einschraubtiefe die Justierschraube 20 die Knochenschraube 5 berührt. Diese zweite Kontaktfläche definiert somit eine mediale Endposition der Knochenschraube 5, ab welcher keine weitere Bewegung der Knochenschraube 5 relativ zum Nagel 1 in Medialrichtung 7, parallel zur Querachse 21, mehr möglich ist, und zwar unabhängig von einer allfälligen weiteren Bewegung der Justierschraube 20 entlang der Justierrichtung 11. Anders ausgedrückt ändert sich eine Position der zweiten Anschlagfläche 10 ab einem Kontakt der Justierschraube 20 mit der Knochenschraube 5 nicht mehr. So ist die mediale Endposition der Knochenschraube 5 ab einem Kontakt der Justierschraube 20 mit der Knochenschraube 5 bei diesem Ausführungsbeispiel festgelegt und ändert sich die mediale Endposition nicht mit einer Einschraubtiefe der Justierschraube 20, wobei diese Justierschraube 20 bei einer Änderung der Einschraubtiefe entlang der Justierrichtung 11 bewegt wird. Eine Position der zweiten Anschlagfläche 10 ist somit bei diesem Ausführungsbeispiel konstruktiv über Abmessungen von Justierschraube 20 und Knochenschraube 5 sowie den Winkel α zwischen Knochenschraube 5 und Justierschraube 20 festgelegt.

Im Unterschied zur zweiten Anschlagfläche 10 ist jedoch eine Position der ersten Anschlagfläche 9 durch eine Änderung der Einschraubtiefe der Justierschraube 20 direkt veränderbar. Die erste Anschlagfläche 9 definiert eine laterale Endposition der Knochenschraube 5, also eine Position, ab welcher keine weitere Bewegung der Knochenschraube 5 in Lateralrichtung 6 relativ zum Nagel 1 mehr möglich ist. Diese erste Anschlagfläche 9 wird hier durch eine Stirnseite der Justierschraube 20 gebildet, welche etwa normal zur Justierachse ausgerichtet ist. Die erste Anschlagfläche 9 kann natürlich alternativ auch durch eine andere Oberfläche der Justierschraube 20 gebildet sein, deren Lage sich durch eine Änderung der Einschraubtiefe relativ zu einer korrespondierenden Fläche der Nut 12 ändert, insbesondere durch eine Gewindeflanke.

Fig. 27 zeigt eine Position der Vorrichtung analog zu der in Fig. 1 dargestellten, wobei eine Einschraubtiefe der Justierschraube 20 maximal ist, sodass die Knochenschraube 5 gleichzeitig sowohl an der ersten Anschlagfläche 9 als auch an der zweiten Anschlagfläche 10 anliegt und kein Spiel 17 besteht, die Knochenschraube 5 somit nicht relativ zum Nagel 1 bewegbar ist.

Fig. 28 zeigt eine Position analog zu der in Fig. 2 oder 3 dargestellten Position, bei welcher die Justierschraube 20 lateral etwas aus dem Nagel 1 geschraubt ist, die Einschraubtiefe also geringer ist als in der in Fig. 27 dargestellten Position. Die Position der Knochenschraube 5 ist gegenüber der in Fig. 27 dargestellten Position unverändert. Nachdem sich die Position der zweiten Anschlagfläche 10 wie ausgeführt hier nicht mit der Einschraubtiefe verändert, liegt die Knochenschraube 5 weiterhin an der zweiten Anschlagfläche 10 an und befindet sich somit weiterhin ein einer medialen Endposition, in welcher keine weitere Bewegung der Knochenschraube 5 in Medialrichtung 7 möglich ist. Allerdings liegt die erste Anschlagfläche 9 nun nicht mehr an der Knochenschraube 5 an, sodass die Knochenschraube 5 relativ zum Nagel 1 entlang der Querachse 21 in Lateralrichtung 6 bewegbar ist. Entsprechend ergibt sich ein Spiel 17 wie dargestellt zwischen der medialen Endposition, in welcher sich die Knochenschraube 5 in Fig. 28 befindet, und einer lateralen Endposition, bis zu welcher die Knochenschraube 5 bewegt werden könnte.

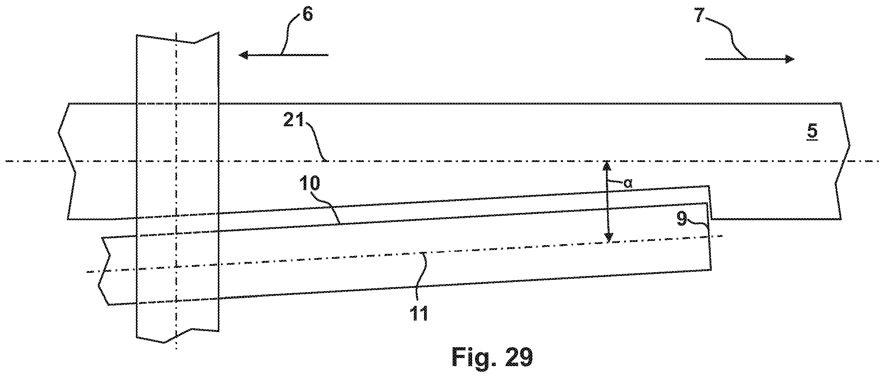

Fig. 29 zeigt die Vorrichtung der Fig. 27 und 28 mit gegenüber der Fig. 28 unveränderter Einschraubtiefe. Im Unterschied zu Fig. 28 befindet sich nun jedoch die Knochenschraube 5 in einer lateralen Endposition und liegt somit an der ersten Anschlagfläche 9 an der Justierschraube 20 an, jedoch nicht mehr an der zweiten Anschlagfläche 10. Entsprechend ist ausgehend von dieser Position nur eine Bewegung der Knochenschraube 5 relativ zum Nagel 1 in Medialrichtung 7, jedoch keine weitere Bewegung in Lateralrichtung 6 möglich.

Eine Änderung der Einschraubtiefe wirkt somit hier mit einem Cosinus des Winkels α, bei kleinen Winkeln α also nahezu 1:1, auf eine Änderung der Position der ersten Anschlagfläche 9, jedoch nicht auf eine Lage der zweiten Anschlagfläche 10. Entsprechend wird auch eine Größe des Spiels 17 nahezu 1:1 mit der Einschraubtiefe verändert.

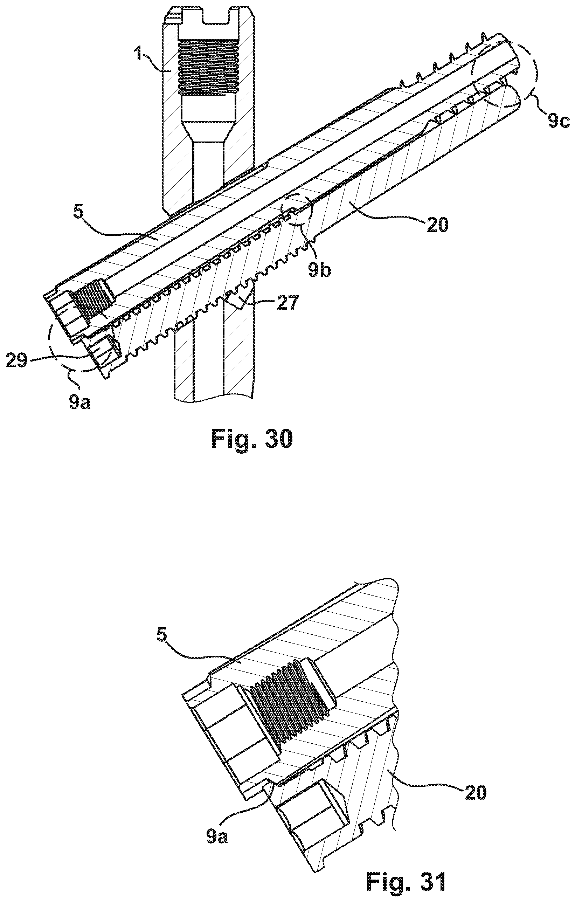

Fig. 30 und 31 zeigen Details eines weiteren Ausführungsbeispiels einer erfindungsgemäßen Vorrichtung. Diese Vorrichtung weist eine durch drei Anschläge, die parallel wirken, gebildete erste Anschlagfläche 9 auf, nämlich einen primären Anschlag 9a an einem lateralen Ende 13 der auch hier durch eine Justierschraube 20 gebildeten Justiereinrichtung, einen sekundären Anschlag 9b an einem medialen Ende 14 eines Gewindes der Justierschraube 20, mit welchem Gewinde die Justierschraube 20 in den Nagel 1 eingeschraubt ist, und einem tertiären Anschlag 9c an einem medialen Ende 14 der Justierschraube 20. Am tertiären Anschlag 9c wirkt ein mediales Ende 14 der Justierschraube 20 mit einem Gewindegang der Knochenschraube 5 zusammen. Durch die drei parallel wirkenden Anschläge 9a, 9b, 9c ist ein unbeabsichtigtes Gleiten der Justierschraube 20 entlang der Knochenschraube 5 auf einfache Weise verhindert und somit eine Bewegbarkeit zuverlässig begrenzt.

Weiter ist diesem Ausführungsbeispiel eine Schraubensicherung vorgesehen, welche durch ein Kunststoffteil 27 in einem Gewindegang im Nagel 1 gebildet wird. Dieses Kunststoffteil 27 erhöht die Reibung zwischen dem Gewinde der Justierschraube 20 und dem Nagel 1, sodass ein unbeabsichtigtes Lösen der Justierschraube 20 verhindert ist.

Die Justierschraube 20 weist hier an einem lateralen Ende 13 einen Innensechskant 29 auf und ist mit einem nicht dargestellten Inbusschlüssel betätigbar. Um ein unbeabsichtigt weites Einschrauben der Justierschraube 20 zu verhindern, ist der Sechskantschlüssel derart ausgebildet, dass dieser nur bis zu einer vordefinierten Position relativ zur Knochenschraube 5 in Medialrichtung 7 entlang der Justierschraube 20 führbar ist. Beispielsweise kann hierzu ein Anschlag am Sechskantschlüssel und/oder der Knochenschraube 5 vorgesehen sein. Dadurch verliert der Inbusschlüssel ab einer vordefinierten Einschraubtiefe der Justierschraube 20 den Kontakt zur Justierschraube 20 bzw. gleitet aus dem Innensechskant 29, sodass kein weiteres Einschrauben möglich ist. Auf diese Weise ist ein unbeabsichtigt weites Einschrauben der Justierschraube 20 bei konstruktiv einfachem Aufbau vermieden.

Fig. 31 zeigt den primären Anschlag im Detail. Wie ersichtlich weist die Justierschraube 20 an einem lateralen ende einen durch einen Justierschraubenkragen 28 gebildeten Kragen auf, welcher ab einer vordefinierten Relativposition von Justierschraube 20 und Knochenschraube 5 an der Knochenschraube 5 anliegt, und somit eine weitere Bewegung der Knochenschraube 5 relativ zur Justierschraube 20 in Lateralrichtung 6 verhindert, sodass dieser primäre Anschlag parallel zum sekundären Anschlag 9b und parallel zum tertiären Anschlag 9c wirkt, sodass ein Gleiten der Justierschraube 20 an der Knochenschraube 5 effektiv vermieden ist.

Ein erfindungsgemäßes Verfahren stellt darüber hinaus sicher, dass kein Knochenzement 26 in die Gelenkskapsel eindringt, wodurch ein Risiko für Komplikationen besonders gering ist.

## Patentansprüche

1. Vorrichtung zum Versorgen einer Fraktur, insbesondere einer Fraktur eines proximalen Femurs wie beispielsweise einer Schenkelhalsfraktur, aufweisend einen Nagel (1), welcher in einen intramedullären Kanal eines Knochens einbringbar ist, insbesondere in einen intramedullären Kanal eines Oberschenkelknochenschaftes (2), wobei der Nagel (1) eine Bohrung (3) aufweist, welche etwa quer zu einer Längsachse (4) des Nagels (1) ausgerichtet ist, wobei eine Koppeleinrichtung, insbesondere eine Knochenschraube (5), vorgesehen ist, welche derart in die Bohrung (3) einbringbar ist, dass die Koppeleinrichtung die Bohrung (3) beidseits überragt und in der Bohrung (3) entlang einer Querachse (21) in einer Lateralrichtung (6) und einer entgegengesetzt zur Lateralrichtung (6) orientierten Medialrichtung (7) bewegbar ist, und welche Koppeleinrichtung endseitig in einem Knochenteil, insbesondere einem Oberschenkelkopf (8), fixierbar ist, insbesondere mittels eines an der Koppeleinrichtung vorgesehenen Gewindes, wobei eine Justiereinrichtung vorgesehen ist, welche in unterschiedlichen Positionen relativ zum Nagel (1) fixierbar ist, insbesondere mittels eines Gewindes in der Justiereinrichtung und im Nagel (1), und wobei die Justiereinrichtung eine erste Anschlagfläche (9) aufweist, welche bei Anordnung der Koppeleinrichtung und der Justiereinrichtung am Nagel (1) eine Bewegbarkeit der Koppeleinrichtung in Lateralrichtung (6) begrenzt, sodass durch eine Änderung der Position der Justiereinrichtung relativ zum Nagel (1) eine laterale Endposition veränderbar ist, bis zu welcher die Koppeleinrichtung relativ zum Nagel (1) in Lateralrichtung (6) bewegbar ist, **dadurch gekennzeichnet, dass** die erste Anschlagfläche (9) durch mehrere Anschlagflächen gebildet ist, welche parallel wirken.

2. Vorrichtung nach Anspruch 1, **dadurch gekennzeichnet, dass** die erste Anschlagfläche (9) zumindest teilweise durch eine Schulter oder einen Kragen, insbesondere einen Justierschraubenkragen (28), in einem Bereich eines lateralen Endes der Justiereinrichtung gebildet ist.

3. Vorrichtung nach Anspruch 1 oder 2, **dadurch gekennzeichnet, dass** die erste Anschlagfläche (9) zumindest teilweise durch einen Teil eines Gewindes der Justiereinrichtung gebildet ist, durch welches Gewinde die Justiereinrichtung relativ zum Nagel (1) fixierbar ist.

4. Vorrichtung nach einem der Ansprüche 1 bis 3, **dadurch gekennzeichnet, dass** die Justiereinrichtung mit dem Nagel durch ein Gewinde verbunden ist, wobei eine Schraubensicherung, insbesondere ein Kunststoffteil, welches eine Reibung im Gewinde erhöht, vorgesehen ist, um ein Lösen der Justiereinrichtung zu verhindern.

5. Vorrichtung nach einem der Ansprüche 1 bis 4, **dadurch gekennzeichnet, dass** die Justiereinrichtung nur bis zu einer vordefinierten Position in den Nagel einschraubbar ist, wobei dies konstruktiv insbesondere mit einem Schraubenschlüssel, vorzugsweise einem Innensechskantschlüssel, umgesetzt ist, der ab einer vordefinierten Position der Justiereinrichtung keinen Kontakt mehr zu einem korrespondierenden Gegenstück, insbesondere einem Innensechskant, in der Justiereinrichtung herstellt.

6. Vorrichtung nach Anspruch 1, **dadurch gekennzeichnet, dass** die Justiereinrichtung eine zweite Anschlagfläche (10) aufweist, welche bei Anordnung der Koppeleinrichtung und der Justiereinrichtung am Nagel (1) eine Bewegbarkeit der Koppeleinrichtung in Medialrichtung (7) begrenzt.

7. Vorrichtung nach Anspruch 1 oder 6, **dadurch gekennzeichnet, dass** eine Position der Justiereinrichtung relativ zum Nagel (1) entlang einer Justierrichtung (11) veränderbar ist, insbesondere entlang einer Geraden.

8. Vorrichtung nach Anspruch 7, **dadurch gekennzeichnet, dass** die erste Anschlagfläche (9) und die zweite Anschlagfläche (10) unter unterschiedlichen Winkeln zur Justierrichtung (11) ausgerichtet sind, sodass bei einer Änderung der Position der Justiereinrichtung relativ zum Nagel (1) die laterale Endposition um einen größeren Betrag als die mediale Endposition änderbar ist.

9. Vorrichtung nach einem der Ansprüche 1 bis 8, **dadurch gekennzeichnet, dass** eine Position der Justiereinrichtung relativ zum Nagel (1) entlang einer Justierrichtung (11) veränderbar ist, wobei die Justierrichtung (11) unter einem Winkel (α) von 0,1° bis 15°, insbesondere 0,8° bis 2°, zur Querrichtung ausgerichtet ist, wobei vorzugsweise die Justierrichtung (11) und die Querrichtung in derselben Ebene liegen.

10. Vorrichtung nach einem der Ansprüche 1 bis 9, **dadurch gekennzeichnet, dass** die Justiereinrichtung länger als die Bohrung (3) ausgebildet ist, sodass die Justiereinrichtung in einer die Bohrung (3) beidseitig überragenden Position mit dem Nagel (1) verbindbar ist, wobei die erste Anschlagfläche (9) medial vom Nagel (1) angeordnet ist.

11. Vorrichtung nach einem der Ansprüche 1 bis 10, **dadurch gekennzeichnet, dass** die Koppeleinrichtung eine mit der Justiereinrichtung zusammenwirkende und längs der Koppeleinrichtung verlaufende Nut (12) aufweist, sodass eine Rotation der Koppeleinrichtung um die Querachse (21) durch die Justiereinrichtung begrenzt, vorzugsweise verhindert, ist, wenn die Koppeleinrichtung und die Justiereinrichtung mit dem Nagel (1) verbunden sind.

12. Vorrichtung nach einem der Ansprüche 1 bis 11, **dadurch gekennzeichnet, dass** die Bohrung (3) zur Aufnahme der Koppeleinrichtung in einem proximalen Bereich und zur Aufnahme der Justiereinrichtung in einem distalen Bereich ausgebildet ist.

13. Vorrichtung nach einem der Ansprüche 1 bis 12, **dadurch gekennzeichnet, dass** die Koppeleinrichtung eine von einem lateralen Ende (13) bis zu einem medialen Ende (14) ragende Durchgangsbohrung (19) aufweist, in welche ein K-Draht (15) einführbar ist.

14. Vorrichtung nach einem der Ansprüche 1 bis 13, **dadurch gekennzeichnet, dass** eine kanülierte Einbringeinrichtung (16) vorgesehen ist, welche alternativ zur Justiereinrichtung mit dem Nagel (1) verbindbar ist, insbesondere anstelle der Justiereinrichtung in die Bohrung (3) des Nagels (1) einführbar ist, sodass durch die kanülierte Einbringeinrichtung (16) Knochenzement (26) bis in einen Bereich eines Oberschenkelkopfes (8) einbringbar ist, wenn der Knochennagel in einem Oberschenkelknochenschaft (2) angeordnet und die Einbringeinrichtung (16) mit dem Nagel (1) verbunden ist.

## Claims

1. An apparatus for treating a fracture, in particular a fracture of a proximal femur, such as a femoral neck fracture, said apparatus comprising a nail (1), which can be introduced into an intramedullary canal of a bone, in particular an intramedullary canal of a femoral bone shaft (2), wherein the nail (1) comprises a bore (3), which is aligned approximately transversely to a longitudinal axis (4) of the nail (1), wherein a coupling device, in particular a bone screw (5), is provided, which can be introduced into the bore (3) in such a way that the coupling device projects beyond the bore (3) on both sides and is movable within the bore (3) along a transverse axis (21) in a lateral direction (6) and a medial direction (7) oriented opposite to the lateral direction (6), and which coupling device is fixable at an end in a bone part, in particular a femoral head (8), in particular by means of a thread provided on the coupling device, wherein an adjusting device is provided, which is fixable in different positions relative to the nail (1), in particular by means of a thread in the adjusting device and in the nail (1), and wherein the adjusting device comprises a first stop surface (9), which, when the coupling device and the adjusting device are arranged on the nail (1), delimits a movability of the coupling device in the lateral direction (6), so that, by changing the position of the adjusting device relative to the nail (1), a lateral end position up to which the coupling device is movable relative to the nail (1) in the lateral direction (6) can be altered, **characterised in that** the first stop surface (9) is formed by a plurality of stop surfaces which act in parallel.

2. The apparatus according to claim 1, **characterised in that** the first stop surface (9) is formed at least in part by a shoulder or a collar, in particular an adjusting screw collar (28), in a region of a lateral end of the adjusting device.

3. The apparatus according to claim 1 or 2, **characterised in that** the first stop surface (9) is formed at least in part by a part of a thread of the adjusting device, by which thread the adjusting device is fixable relative to the nail (1).

4. The apparatus according to any one of claims 1 to 3, **characterised in that** the adjusting device is connected to the nail by a thread, wherein a thread lock, in particular a plastics part, which increases friction in the thread, is provided in order to prevent a loosening of the adjusting device.

5. The apparatus according to any one of claims 1 to 4, **characterised in that** the adjusting device can be screwed into the nail only up to a predefined position, wherein this is implemented constructively in particular with a wrench, preferably a hex wrench, which, from a predefined position of the adjusting device, no longer produces any contact with a corresponding counterpiece, in particular a hex socket, in the adjusting device.

6. The apparatus according to claim 1, **characterised in that** the adjusting device comprises a second stop surface (10), which, when the coupling device and the adjusting device are arranged on the nail (1), delimits a movability of the coupling device in the medial direction (7).

7. The apparatus according to claim 1 or 6, **characterised in that** the position of the adjusting device relative to the nail (1) can be altered along an adjusting direction (11), in particular along a straight line.

8. The apparatus according to claim 7, **characterised in that** the first stop surface (9) and the second stop surface (10) are aligned at different angles to the adjusting direction (11), so that, when the position of the adjusting device relative to the nail (1) is changed, the lateral end position can be changed by a greater magnitude than the medial end position.

9. The apparatus according to any one of claims 1 to 8, **characterised in that** the position of the adjusting device relative to the nail (1) can be altered along an adjusting direction (11), wherein the adjusting direction (11) is aligned at an angle (α) of from 0.1° to 15°, in particular 0.8° to 2°, to the transverse direction, wherein preferably the adjusting direction (11) and the transverse direction lie in the same plane.

10. The apparatus according to any one of claims 1 to 9, **characterised in that** the adjusting device is longer than the bore (3), so that the adjusting device is connectable to the nail (1) in a position projecting beyond the bore (3) on both sides, wherein the first stop surface (9) is arranged medially from the nail (1).

11. The apparatus according to any one of claims 1 to 10, **characterised in that** the coupling device comprises a groove (12) that interacts with the adjusting device and runs along the coupling device, so that rotation of the coupling device about the transverse axis (21) is delimited by the adjusting device, preferably prevented, when the coupling device and the adjusting device are connected to the nail (1).

12. The apparatus according to any one of claims 1 to 11, **characterised in that** the bore (3) is designed to receive the coupling device in a proximal region and to receive the adjusting device in a distal region.

13. The apparatus according to any one of claims 1 to 12, **characterised in that** the coupling device comprises a through-bore (19) projecting from a lateral end (13) to a medial end (14), into which through-bore a K-wire (15) can be inserted.

14. The apparatus according to any one of claims 1 to 13, **characterised in that** a cannulated introduction device (16) is provided, which, as an alternative to the adjusting device, is connectable to the nail (1), in particular can be inserted instead of the adjusting device into the bore (3) of the nail (1), so that bone cement (26) can be introduced up to a region of a femoral head (8) through the cannulated introduction device (16) when the bone nail is arranged in a femoral bone shaft (2) and the introduction device (16) is connected to the nail (1).

## Revendications

1. Dispositif permettant de soigner une fracture, en particulier une fracture d'un fémur proximal, telle qu'une fracture du col du fémur, présentant une broche (1) qui peut être insérée dans un canal intramédullaire d'un os, en particulier dans un canal intramédullaire d'une tige fémorale (2), la broche (1) comportant un alésage (3) qui est orienté approximativement transversalement par rapport à un axe longitudinal (4) de la broche (1), étant prévu un dispositif de couplage, en particulier une vis osseuse (5), qui peut être inséré dans l'alésage (3) de manière à ce que le dispositif de couplage dépasse de l'alésage (3) des deux côtés et qu'il soit mobile dans l'alésage (3) le long d'un axe transversal (21) dans une direction latérale (6) et dans une direction médiale (7) orientée à l'opposé de la direction latérale (6), et lequel dispositif de couplage pouvant être fixé en extrémité dans une partie osseuse, en particulier une tête de fémur (8), notamment au moyen d'un filetage prévu sur le dispositif de couplage, étant prévu un dispositif de réglage qui peut être fixé dans différentes positions par rapport à la broche (1), notamment au moyen d'un filetage dans le dispositif de réglage et la broche (1), et le dispositif de réglage présentant une première surface de butée (9) qui, lorsque le dispositif de couplage et le dispositif de réglage sont disposés sur la broche (1), limite la mobilité du dispositif de couplage dans la direction latérale (6), de sorte qu'une modification de la position du dispositif de réglage par rapport à la broche (1) permet de modifier une position finale latérale jusqu'à laquelle le dispositif de couplage peut être déplacé dans la direction latérale (6) par rapport à la broche (1), **caractérisé en ce que** la première surface de butée (9) est formée par plusieurs surfaces de butée qui agissent en parallèle.

2. Dispositif selon la revendication 1, **caractérisé en ce que** la première surface de butée (9) est formée au moins en partie par un épaulement ou une collerette, en particulier une collerette à vis de réglage (28), dans une zone d'une extrémité latérale du dispositif de réglage.

3. Dispositif selon la revendication 1 ou 2, **caractérisé en ce que** la première surface de butée (9) est formée au moins en partie par une partie d'un filetage du dispositif de réglage, par lequel filetage le dispositif de réglage peut être fixé par rapport à la broche (1).

4. Dispositif selon l'une des revendications 1 à 3, **caractérisé en ce que** le dispositif de réglage est relié à la broche par un filetage, étant prévue une sécurité à vis, en particulier une pièce en plastique qui augmente le frottement dans le filetage pour éviter le desserrage du dispositif de réglage.

5. Dispositif selon l'une des revendications 1 à 4, **caractérisé en ce que** le dispositif de réglage ne peut être vissé dans la broche que jusqu'à une position prédéfinie, en particulier avec une clé à vis, de préférence une clé à six pans creux, qui, à partir d'une position prédéfinie du dispositif de réglage, n'établit plus de contact avec une contrepartie correspondante, en particulier une pièce à six pans creux, dans le dispositif de réglage.

6. Dispositif selon la revendication 1, **caractérisé en ce que** le dispositif de réglage présente une seconde surface de butée (10) qui, lorsque le dispositif de couplage et le dispositif de réglage sont disposés sur la broche (1), limite la mobilité du dispositif de couplage dans la direction médiale (7).

7. Dispositif selon la revendication 1 ou 6, **caractérisé en ce qu'**une position du dispositif de réglage par rapport à la broche (1) peut être modifiée le long d'un sens de réglage (11), en particulier le long d'une ligne droite.

8. Dispositif selon la revendication 7, **caractérisé en ce que** la première surface de butée (9) et la seconde surface de butée (10) sont alignées sous des angles différents par rapport au sens de réglage (11), de sorte que, si la position du dispositif de réglage est modifiée par rapport à la broche (1), la position finale latérale peut être modifiée d'une amplitude supérieure à la position finale médiale.

9. Dispositif selon l'une des revendications 1 à 8, **caractérisé en ce qu'**une position du dispositif de réglage par rapport à la broche (1) peut être modifiée le long d'un sens de réglage (11), le sens de réglage (11) étant orienté par rapport à la direction transversale suivant un angle (α) de 0,1° à 15°, en particulier de 0,8° à 2°, le sens de réglage (11) et le sens transversal se trouvant de préférence dans le même plan.

10. Dispositif selon l'une des revendications 1 à 9, **caractérisé en ce que** le dispositif de réglage a une conformation plus longue que l'alésage (3), de sorte que le dispositif de réglage peut être relié à la broche (1) dans une position dépassant de l'alésage (3) des deux côtés, la première surface de butée (9) étant disposée médialement par rapport à la broche (1).

11. Dispositif selon l'une des revendications 1 à 10, **caractérisé en ce que** le dispositif de couplage comporte une rainure (12) en interaction avec le dispositif de réglage et s'étendant longitudinalement par rapport au dispositif de couplage, de sorte que la rotation du dispositif de couplage autour de l'axe transversal (21) est limitée, de préférence empêchée, par le dispositif de réglage lorsque le dispositif de couplage et le dispositif de réglage sont connectés à la broche (1).

12. Dispositif selon l'une des revendications 1 à 11, **caractérisé en ce que** l'alésage (3) est conçu pour accueillir le dispositif de couplage dans une zone proximale et pour accueillir le dispositif de réglage dans une zone distale.

13. Dispositif selon l'une des revendications 1 à 12, **caractérisé en ce que** le dispositif de couplage comporte un trou de passage (19), qui s'étend d'une extrémité latérale (13) à une extrémité médiale (14) et dans lequel un fil K (15) peut être inséré.

14. Dispositif selon l'une des revendications 1 à 13, **caractérisé en ce qu'**il est prévu un dispositif d'insertion canulé (16) qui peut être relié à la broche (1) en remplacement du dispositif de réglage, notamment inséré dans l'alésage (3) de la broche (1) en lieu et place du dispositif de réglage, de sorte que du ciment osseux (26) puisse être inséré dans une zone de la tête de la cuisse (8) par le dispositif d'insertion canulé (16) lorsque la broche osseuse est placée dans une tige de fémur (2) et que le dispositif d'insertion (16) est connecté à la broche (1).
